# EUROPEAN PATENT APPLICATION

(11) **EP 4 772 883 A1**
(43) Date of publication of application: **08.07.2026**
(21) Application number: 24859678.5
(22) Date of filing: 26.08.2024
(51) Int. Cl.: G01N 33/544, G01N 33/543

(54) **METHOD FOR SIMULTANEOUS DETECTION OF BIOMOLECULES USING TEMPERATURE-RESPONSIVE FLUORESCENT PARTICLES**

(30) Priority: 29.08.2023 JP 2023139373
(71) Applicant: WASEDA UNIVERSITY, Shinjuku-ku Tokyo 169-8050 (JP)
(72) Inventor: TAKEOKA, Shinji, Tokyo 169-8050 (JP); SOU, Keitaro, Tokyo 169-8050 (JP); MARUYAMA, Taro, Tokyo 169-8050 (JP)
(74) Representative: J A Kemp LLP
(86) International application number: PCT/JP2024/030148
(87) International publication number: WO 2025/047645

(57) **Abstract**

The present invention aims to provide a method for simultaneously detecting multiple target biomolecules in a single assay using temperature-responsive fluorescent particles. The above-mentioned problem is solved by providing a method for detecting or quantifying a biomolecule in a biological sample, including the following steps (1) to (3):
(1) a step of bringing a magnetic particle bound to a biomolecular recognition element for capture, a biological sample, and a temperature-responsive fluorescent probe particle into contact with a magnetic substrate,
(2) a step of heating the aforementioned magnetic substrate, and
(3) a step of measuring fluorescence emission.

## Description

### [Technical Field]

The present invention relates to a method for simultaneously detecting biomolecules by using temperature-responsive fluorescent particles.

### [Background Art]

In recent years, ELISA method has been frequently used for the detection and quantification of biomolecules (biomarkers). Since ELISA method utilizes an antigen-antibody reaction, high specificity is achieved. On the other hand, the operations of washing reagents, enzyme reactions, and the like are complicated and time-consuming, and the detection concentration of biomolecules is limited to about 1 pM.

Furthermore, biomolecule detection methods that further advance the ELISA method have also been developed. For example, a method called digital ELISA method achieves high sensitivity by performing an enzyme reaction in a microdroplet (femtoliter) by using a devised reaction device (Non Patent Literatures 1 and 2). However, the measurement principle of these methods is the same as that of the ELISA method, and they problematically require more complicated and time-consuming operations than conventional ELISA method.

Single Molecule Counting (SMC(trademark)) Immunoassay Technology has been developed as a molecule detection technique (Non Patent Literature 3). This technique is also based on the principle of ELISA method, but the detection area is made smaller by improving the optical system of the detector, thus improving the detection sensitivity. However, it requires a dedicated, expensive detector, and the measurement takes time because a small area is repeatedly scanned and measured.

Furthermore, immunochromatography has been used (Non Patent Literature 4), but it has drawbacks such as low sensitivity that prevents use for early detection of disease or infection, differences in visual sensitivity among examiners who make visual judgment, and a high false negative rate (20 to 30%).

The present inventors have previously reported that they have developed temperature-responsive fluorescent particles whose fluorescence intensity changes depending on the temperature, and have succeeded in developing a method for detecting target biomolecules (biomarkers) with low concentrations in a short time using the particles (Patent Literature 1). However, this method has a problem in that it needs to be performed individually for each target biomolecule contained in a biological sample, and is not suitable for simultaneous detection of multiple target biomolecules in a single assay.

### [Citation List]

### [Patent Literature]

[Patent Literature 1]
JP-B- 7284470

### [Non Patent Literature]

[Non Patent Literature 1]
   Rissin D.M., et al, Nat Biotechnol. 2010:28(6):595-9.
[Non Patent Literature 2]
   Kim S.H., et al, Lab Chip, 2012, 12, 4986-4991.
[Non Patent Literature 3]
   Hwang J., et al, Methods, 2019, 158, 69-79.
[Non Patent Literature 4]
   Hurt C. A. et al, J. Clin. Virol., 2007, 39, 132-135

### [Summary of Invention]

### [Technical Problem]

The problem is to provide a method for simultaneously detecting multiple target biomolecules in a single assay using temperature-responsive fluorescent particles.

### [Solution to Problem]

The present inventors confirmed that a lipid vesicle (temperature-responsive fluorescent particle) containing a fluorescent squaric acid derivative represented by the following formula I: 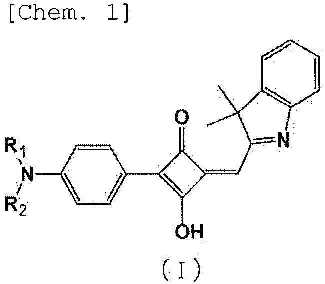
wherein R₁ and R₂ are each independently the same or different hydrophobic group,
as a fluorescent molecule shows a stepwise increase in fluorescence intensity depending on the heating time when lipid vesicles with different lipid acyl chain lengths are mixed and heated. Thus, the present inventors brought multiple magnetic particles having, on their surface, antibodies that target respective detection targets, multiple temperature-responsive fluorescent particles (temperature-responsive fluorescent probe particles that emit fluorescence at mutually different temperatures) having, on their surface, antibodies that target respective detection targets and having different lipid acyl chain lengths, and biological samples into contact with each other on the same magnetic substrate, heated them, and observed changes in fluorescence intensity over time. As a result, they confirmed that the presence or absence of each detection target corresponds to heating time-dependent changes in fluorescence intensity, and confirmed that the range of change in fluorescence intensity correlates to the concentration of the detection target, which resulted in the completion of the present invention.

Accordingly, the present invention provides the following.
[1] A method for detecting or quantifying a biomolecule in a biological sample, comprising the following steps (1) to (3):
   (1) a step of bringing a magnetic particle bound to a biomolecular recognition element for capture, a biological sample, and a temperature-responsive fluorescent probe particle into contact with a magnetic substrate,
   (2) a step of heating the aforementioned magnetic substrate, and
   (3) a step of measuring fluorescence emission, wherein
      the aforementioned temperature-responsive fluorescent probe particle is a temperature-responsive fluorescent particle bound to a biomolecular recognition element for detection,
      the aforementioned temperature-responsive fluorescent particle comprises at least one type of fluorescent molecule in a molecular assembly comprising and constituted by at least one type of amphiphilic lipid molecule,
      the aforementioned fluorescent molecule is quenched when the aforementioned molecular assembly is in a solid phase, and emits fluorescence when the assembly is in a liquid phase, due to a temperature-responsive solid-liquid phase transition,
      whereby quenching and fluorescence emission of the fluorescent molecule are reversibly switched in response to temperature,
      the aforementioned molecular assembly comprises aggregated fluorescent molecules when it is in a solid phase and dissociated fluorescent molecules when it is in a liquid phase, and
      the aforementioned fluorescent molecule is at least one compound selected from the group consisting of squaric acid derivatives, fluorescein derivatives, rhodamines, coumarins, oxazines, pyrenes, anthracenes, phthalocyanines, cyanines, azo dyes, carbopyronines, NBD derivatives, dansyl derivatives, BODIPY fluorophores, ALEXA fluorophores, rhodol dyes, naphthalimides, and porphyrins.
[2] The method for detecting or quantifying a biomolecule of [1], wherein the aforementioned step (1) comprises the following steps:
   (1') a step of bringing a magnetic particle bound to a biomolecular recognition element for capture into contact with a magnetic substrate,
   (2') a step of contacting a biological sample with the aforementioned magnetic particle, and
   (3') a step of contacting a temperature-responsive fluorescent probe particle with the aforementioned magnetic particle.
[3] The method for detecting or quantifying a biomolecule of [1], wherein the aforementioned step (1) comprises the following steps:
   (1") a step of bringing a magnetic particle bound to a biomolecular recognition element for capture into contact with a biological sample,
   (2") a step of contacting a complex obtained in step (1") with a magnetic substrate,
   (3") a step of contacting a temperature-responsive fluorescent probe particle with the aforementioned complex, and
   (4") a step of contacting the complex obtained in step (3") with the magnetic substrate.
[4] The method for detecting or quantifying a biomolecule of [1], wherein the aforementioned step (1) comprises the following steps:
   (1‴) a step of contacting a temperature-responsive fluorescent probe particle with a biological sample,
   (2‴) a step of contacting a complex obtained in step (1‴) with a magnetic particle bound to a biomolecular recognition element for capture, and
   (3‴) a step of contacting the complex obtained in step (2‴) with a magnetic substrate.
[5] The method for detecting or quantifying a biomolecule of any one of [1] to [4], comprising a step of washing the aforementioned magnetic particle between the aforementioned steps (1) and (2).
[6] The method for detecting or quantifying a biomolecule of any one of [1] to [5], wherein the aforementioned biomolecules to be detected or quantified are two or more types,
   the aforementioned magnetic particles are two or more types of magnetic particles bound to mutually different biomolecular recognition elements for capture, and
   the aforementioned temperature-responsive fluorescent probe particles are two or more types of temperature-responsive fluorescent particles bound to mutually different biomolecular recognition elements for detection and having different solid phase-liquid phase transition temperatures.
[7] The method for detecting or quantifying a biomolecule of any one of [1] to [6], wherein the aforementioned molecular assembly has a lipid bilayer, and the aforementioned fluorescent molecule is quenched when the aforementioned molecular assembly is in a gel phase, and emits fluorescence when the assembly is in a liquid crystalline phase, due to a temperature-responsive gel-liquid crystalline phase transition,
   whereby quenching and fluorescence emission of the fluorescent molecule are reversibly switched in response to temperature.
[8] The method for detecting or quantifying a biomolecule of any one of [1] to [7], wherein the aforementioned amphiphilic molecule is a phospholipid.
[9] The method for detecting or quantifying a biomolecule of [8], wherein the aforementioned molecular assembly is a bilayer membrane vesicle.
[10] The method for detecting or quantifying a biomolecule of [9], wherein the aforementioned bilayer membrane vesicle comprises 0.3 mol % or more of a compound represented by the aforementioned formula I.
[11] The method for detecting or quantifying a biomolecule of any one of [1] to [10], wherein the aforementioned biomolecular recognition element is an antibody or a variable region of the antibody or a fab fragment of the antibody.
[12] A kit for detecting or quantifying a biomolecule in a biological sample, comprising the following (1) and (2):
   (1) a magnetic particle bound to a biomolecular recognition element for capture, and
   (2) a temperature-responsive fluorescent probe particle which is a temperature-responsive fluorescent particle bound to a biomolecular recognition element for detection,
      the aforementioned temperature-responsive fluorescent particle comprises at least one type of fluorescent molecule in a molecular assembly comprising and constituted by at least one type of amphiphilic lipid molecule,
      the aforementioned fluorescent molecule is quenched when the aforementioned molecular assembly is in a solid phase, and emits fluorescence when the assembly is in a liquid phase, due to a temperature-responsive solid-liquid phase transition,
      whereby quenching and fluorescence emission of the fluorescent molecule are reversibly switched in response to temperature,
      the aforementioned molecular assembly comprises aggregated fluorescent molecules when it is in a solid phase and dissociated fluorescent molecules when it is in a liquid phase, and
      the aforementioned fluorescent molecule is at least one compound selected from the group consisting of squaric acid derivatives, fluorescein derivatives, rhodamines, coumarins, oxazines, pyrenes, anthracenes, phthalocyanines, cyanines, azo dyes, carbopyronines, NBD derivatives, dansyl derivatives, BODIPY fluorophores, ALEXA fluorophores, rhodol dyes, naphthalimides, and porphyrins.
[13] The kit for detecting or quantifying a biomolecule of [12], further comprising a microfluidic chip.

### [Advantageous Effects of Invention]

According to the present invention, multiple target biomolecules contained in a biological sample can be simultaneously detected or quantified in a single assay, and a large amount of target biomolecules can be treated in a shorter time than before.

### [Brief Description of Drawings]

Fig. 1 is a diagram showing the structural formula of SQR22.
Fig. 2A is a diagram showing the ultraviolet visible absorption spectrum of SQR23 (ethanol solution).
Fig. 2B is a diagram showing the fluorescence emission spectrum of SQR23 (ethanol solution).
Fig. 3A is a schematic diagram showing the temperature-responsive liposome containing SQR dye.
Fig. 3B is a diagram showing the fluorescence emission property of temperature-responsive fluorescent liposomes with different phase transition temperatures.
Fig. 4 is a diagram showing the temperature-responsive fluorescence emission property of B-TLip.
Fig. 5 is a diagram showing the time course changes in fluorescence intensity when B-TLip dispersion was heated on a hot plate.
Fig. 6A is a schematic diagram showing the detection of binding by biotin-streptavidin molecular recognition using temperature-responsive liposomes (TLips) and magnetic particles.
Fig. 6B is a diagram showing the detection of binding by biotin-streptavidin molecular recognition using temperature-responsive liposomes (TLips) and magnetic particles. The negative control used was magnetic particles without streptavidin modification, and the other was streptavidin-modified magnetic particles blocked with different amounts of biotin and then bound to biotinylated TLiP.
Fig. 7 is a schematic diagram showing the antigen detection by antigen-antibody sandwich using temperature-responsive liposome (TLip) and magnetic particles.
Fig. 8 is a diagram showing the effect of reaction time in the antigen detection by antigen-antibody sandwich using temperature-responsive liposome (TLip) and magnetic particles.
Fig. 9 is a design diagram showing a microfluidic device for detection.
Fig. 10 is a schematic diagram showing the injection and flow of solutions into a microfluidic device for detection.
Fig. 11 is a diagram showing the process of antigen detection in a microfluidic device using a detection antibody-labeled IgG-TLip and capture antibody-labeled magnetic particles.
Fig. 12 is a diagram showing the time course changes in fluorescence intensity when IgG-TLip bound to magnetic particles via an antigen was captured with a magnet and heated.
Fig. 13A is a diagram showing the the results of detection of SARS-CoV-2 recombinant nucleocapsid protein (N antigen). NC indicates negative control.
Fig. 13B is a diagram showing a plot of the logarithm of N antigen concentration versus the difference in fluorescence intensity before and after heating.
Fig. 14A is a schematic diagram showing simultaneous detection of heterologous antigens using temperature-responsive fluorescent liposomes with different gel-liquid crystalline phase transition temperatures (emission temperatures).
Fig. 14B is a schematic diagram showing a waveform pattern in simultaneous detection of heterologous antigens using temperature-responsive fluorescent liposomes with different gel-liquid crystalline phase transition temperatures (emission temperatures).
Fig. 15 is a diagram showing time course changes in the fluorescence value of each of B-TLip (DMPC), B-TLip (DPPC), and a mixture thereof, which have different gel-liquid crystalline phase transition temperatures, when heated from room temperature.
Fig. 16 is a diagram showing detection based on difference in emission time of B-TLip (DMPC), B-TLip (DPPC), and a mixture thereof, which are bound to streptavidin-modified magnetic particles.
Fig. 17A is a diagram showing detection of influenza A recombinant nucleocapsid protein (N antigen) using an influenza A detection reagent (DMPC) and confirmation of interference action due to the coexistence with a SARS-CoV-2 detection reagent (DPPC).
Fig. 17B is a diagram showing detection of influenza A recombinant nucleocapsid protein (N antigen) using an influenza A detection reagent (DMPC) and confirmation of interference action due to the coexistence with a SARS-CoV-2 N antigen.
Fig. 18 is a diagram showing time course changes in the fluorescence intensity when magnetic particles bound to an influenza A capture antibody and magnetic particles bound to a SARS-CoV-2 capture antibody were mixed, and the mixture was treated with (a) influenza A antigen only, (b) SARS-CoV-2 antigen only, (c) both influenza A antigen and SARS-CoV-2 antigen, or (d) no antigen, treated with both influenza A detection reagent (DMPC) and SARS-CoV-2 detection reagent (DPPC), and then heated (each condition was performed three times).
Fig. 19 is a diagram showing time course changes in the fluorescence intensity when influenza A detection reagent (DMPC) and SARS-CoV-2 detection reagent (DPPC) bound to magnetic particles via N antigens of influenza A and SARS-CoV-2 were heated.
Fig. 20A is a diagram showing simultaneous detection of influenza A and SARS-CoV-2 N antigens using an influenza A detection reagent (DMPC) and a SARS-CoV-2 detection reagent (DPPC). It shows changes in the differential fluorescence intensity when the concentration of SARS-CoV-2 N antigen was fixed and the concentration of influenza A N antigen was changed.
Fig. 20B is a diagram showing simultaneous detection of influenza A and SARS-CoV-2 N antigens using an influenza A detection reagent (DMPC) and a SARS-CoV-2 detection reagent (DPPC). It shows changes in the differential fluorescence intensity when the concentration of influenza A N antigen was fixed and the concentration of SARS-CoV-2 N antigen was changed.
Fig. 21 is a diagram showing simultaneous detection of influenza A and SARS-CoV-2 N antigens on a microfluidic device.

### [Description of Embodiments]

The present invention provides a method for detecting or quantifying a biomolecule in a biological sample (hereinafter the method for detecting or quantitatively determining a biomolecule of the present invention).

Examples of the biological sample to be used in the aforementioned method for detecting or quantitatively determining a biomolecule of the present invention include samples derived from animals or cells. The aforementioned sample may be a sample known to contain a target biomolecule, or may be a sample which is not known to contain or not contain the target biomolecule. Examples of the animal or cell include mammals such as mouse, rat, hamster, guinea pig, dog, monkey, orangutan, chimpanzee, and human, and cells derived from such animals. Examples of the sample derived from an animal include blood, serum, plasma, saliva, urine, tears, sweat, milk, nasal drip, semen, breast water, gastrointestinal secretion, cerebrospinal fluid, interstitial fluid, nasal swab, throat swab, lymph fluid, and the like, and blood, serum, and plasma are preferred. Examples of cell-derived samples include proteins, nucleic acids (DNA, RNA), and the like contained within cells or cell membranes obtained by disrupting cell membranes. These samples can be obtained by a method known per se. For example, serum and plasma can be prepared by collecting blood from an animal according to a conventional method and separating fluid components. Cerebrospinal fluid can be collected by a known means such as spinal puncture. Nasal swab and throat swab can be obtained by a known method from the mucosal walls of the nasal cavity or pharynx using a cotton swab. Cells can be disrupted by treating according to a conventional method (e.g., freeze-thawing, ultrasonication, surfactant treatment, and the like) to recover proteins and nucleic acids (DNA, RNA).

The method for detecting or quantifying a biomolecule of the present invention includes the following steps:
(1) a step of bringing a magnetic particle bound to a biomolecular recognition element for capture, a biological sample, and a temperature-responsive fluorescent probe particle into contact with a magnetic substrate (step (1) of the present invention),
(2) a step of heating the aforementioned magnetic substrate (step (2) of the present invention),
(3) a step of measuring fluorescence emission (step (3) of the present invention).

In step (1) of the present invention, magnetic particles bound to a biomolecular recognition element for capture, a biological sample, and temperature-responsive fluorescent probe particles are brought into contact with a magnetic substrate.

In step (1) of the present invention, the magnetic particles are not particularly limited as long as they are particles having magnetism, and examples include metal particles and magnetic beads. The particle diameter of the magnetic particles is generally 1 µm to 10 µm. The surface of the magnetic particles may also be coated with a hydrophilic polymer to allow the introduction of reactive functional groups. Examples of the reactive functional group include maleimide group, thiol group, amino group, hydroxy group, carboxy group, propargyl group, azido group and the like.

A biomolecular recognition element for capture is bound to magnetic particles. In the present specification, the "element" means a molecule that exerts a specific function such as a biomolecule recognition function, and the "element" and "molecule" can be used interchangeably. In the present specification, the "recognition" means recognizing a target molecule and binding to the molecule. The "biomolecular recognition element" includes not only an element that in itself directly recognizes a biomolecule, but also an element that indirectly recognizes a biomolecule through binding to other molecules or molecular complexes (such as a complex of streptavidin and a biotinylated antibody that recognizes a biomolecule).

Examples of the aforementioned biomolecular recognition element include antibody, the variable region of said antibody, and the Fab fragment of said antibody (hereinafter indicated as "antibody, etc."). In the present specification, an element, such as an antibody, for an epitope of the target biomolecule is sometimes used to adhere the target biomolecule on a magnetic particle. Such element is referred to as a "biomolecular recognition element for capture", and when the element is an antibody or the like, it is also referred to as a "capture antibody". On the other hand, an element, such as an antibody, for another epitope of the target biomolecule recognized by the biomolecular recognition element for capture may be used. In the present specification, such element is referred to as a "biomolecular recognition element for detection", and when the element is an antibody or the like, it is also referred to as a "detection antibody". The above-mentioned "another epitope" may be any epitope on the target biomolecule other than the epitope that the "biomolecular recognition element for capture" actually binds to. Therefore, for example, when there are multiple epitopes of the same type on a target biomolecule, epitopes of the same type other than the epitope to which the "biomolecular recognition element for capture" actually binds are included in the above-mentioned "other epitope". Other examples of the aforementioned biomolecular recognition element include, but are not limited to, a combination of a sugar chain and lectin, a complementary base sequence of nucleic acid, a combination of a receptor and a ligand molecule, a nucleic acid aptamer, and a peptide aptamer.

The magnetic particle and the biomolecular recognition element for capture are bound by a chemical reaction between reactive functional groups on the surface of the magnetic particle and reactive functional groups on the biomolecular recognition element for capture. For example, magnetic particles and biomolecular recognition elements for capture are linked by combinations such as maleimide group and thiol group, amino group or hydroxyl group, and carboxyl group, propargyl group and azide group, and the like. Magnetic particles and biomolecular recognition elements for capture can also be linked via non-covalent bonds and, for example, magnetic particles with streptavidin bound to the surface can be mixed with a biomolecular recognition element for capture with biotin chemically bound thereto, or conversely, magnetic particles with biotin bound to the surface can be mixed with a biomolecular recognition element for capture with streptavidin chemically bound thereto.

In step (1) of the present invention, the temperature-responsive fluorescent probe particle is a temperature-responsive fluorescent particle bound to a biomolecular recognition element for detection.

The temperature-responsive fluorescent particle contains at least one type of fluorescent molecule in a molecular assembly containing and constituted by at least one type of amphiphilic molecule. That is, the present invention utilizes the phenomenon that a quenching state due to aggregation of fluorescent molecules is dissociated into an emitting state, along with thermal transition (that is, a transition from a solid phase to a liquid phase, or a transition from a gel phase to a liquid crystalline phase). Therefore, the temperature-responsive fluorescent particle used in the present invention can be used without particular limitation as long as the phase transition occurs from a solid phase state to a liquid phase state while maintaining the form of the molecular assembly. The aforementioned molecular assembly preferably has a monolayer membrane or a bilayer membrane, more preferably a lipid bilayer membrane (also referred to as a lipid bilayer).

The aforementioned amphiphilic molecule (e.g., lipid molecule) is not limited to lipids derived from living organisms, but includes small molecules or derivatives thereof, such as phospholipid and the like, produced by semisynthesis or synthesis, and amphiphilic molecules that can be synthetically produced using macromolecules, peptides, amino acids, carboxylic acids, and the like as raw materials.

In the aforementioned temperature-responsive fluorescent particle, the aforementioned fluorescent molecules are dissociated and emit fluorescence when the aforementioned molecular assembly is in the liquid phase, and the fluorescent molecules are aggregated and quenched when the molecular assembly is in the solid phase, due to a temperature-responsive solid-liquid phase transition, so that fluorescence emission and quenching of the fluorescent molecules are reversibly switched in response to temperature.

That is, the aforementioned molecular assembly exhibits a temperature-responsive solid-liquid phase transition, in which the molecular assembly contains fluorescent molecules in a dissociated form which emit fluorescence when the molecular assembly is in the liquid phase, and the molecular assembly contains fluorescent molecules in an aggregated form which are quenched due to aggregation-caused quenching (ACQ) when the molecular assembly is in the solid phase. When the aforementioned molecular assembly is a molecular assembly having a lipid bilayer, the aforementioned solid phase is a gel phase and the liquid phase is a liquid crystalline phase.

The aforementioned fluorescent molecule is suitably an amphiphilic or liposoluble fluorescent molecule, because it is arranged in a hydrophobic region in which the fluorescent molecule is constructed in molecular assembling state in the aqueous phase, for example, in a region surrounded by a monolayer membrane or in a bilayer membrane such as a lipid bilayer membrane. Since the fluorescent molecule generally has the property of quenching by aggregation, any fluorescent molecule that exhibits aggregation and dissociation in response to a solid-liquid phase transition (e.g., gel-liquid crystalline phase transition of lipid bilayer membrane) can be switched between quenching and emission.

Many fluorescent molecules that exhibit fluorescence emission exhibit fluorescence emission by emitting energy as light from an excited state and returning to a ground state in dilute solutions. However, the luminescence efficiency drops significantly in highly concentrated solutions or solids. This decrease in luminescence efficiency is considered to result from chaotic energy transfer in the excited state due to aggregation of fluorescent molecules, or from thermal dissipation of energy (non-radiative deactivation) due to interactions between excitons and with molecules in the ground state. It is known as aggregation-caused quenching (ACQ).

π-Conjugated compounds, which are highly planar and have extended π-conjugated systems, have high aggregation property due to strong intermolecular π-π stacking, and many fluorescent molecules are known to exhibit ACQ. Reference documents 1 - 8 describe that a wide variety of fluorescent molecules, such as squaric acid derivatives, fluorescein derivatives, rhodamines, coumarins, oxazines, pyrenes, anthracenes, phthalocyanines, cyanines, BODIPY fluorophores, naphthalimides, and porphyrins, quench due to aggregation. While ACQ has conventionally been a problem for fluorescent molecules, the present invention utilizes the ACQ property of fluorescent molecules, which occurs when molecular assemblies in an aqueous phase transition from a quenched state in the solid phase (including the gel phase) to the liquid phase (including the liquid crystalline phase). Therefore, any of a variety of conventional fluorescent molecules known to exhibit ACQ can be used.

As a method for supporting these fluorescent molecules in a molecular assembly in the aqueous phase, highly hydrophobic fluorescent chromophores can be directly introduced into the hydrophobic portion of molecular assemblies formed by amphiphilic molecules (e.g., lipid bilayer membrane). In another embodiment, various fluorescent chromophores can be incorporated into lipid molecular assemblies formed by amphiphilic molecules by chemically linking a hydrophobic group to the fluorescent chromophore, as shown in the following formula (A) (Reference documents 9 - 12).

In one embodiment, examples of the fluorescent molecule include a compound represented by the following formula (A).

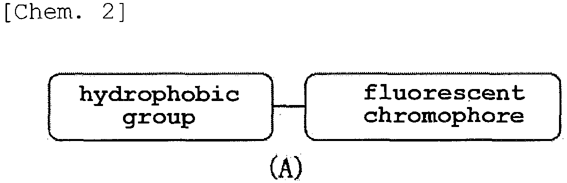

In the formula (A), the hydrophobic group is not particularly limited as long as it can be linked to the fluorescent chromophore via a covalent bond. Examples include straight chain saturated hydrocarbon, straight chain unsaturated hydrocarbon, branched saturated hydrocarbon, branched unsaturated hydrocarbon, saturated fatty acid, unsaturated fatty acid, diacylglycerol, diacylphosphatidylethanolamine, N,N-dialkylaniline, aromatic hydrocarbon, amino acid derivative, sterols, terpenes, and the like. Examples of the structures thereof are shown below. In the following formula, F represents a fluorescent chromophore, and n is an integer of 1 or more.

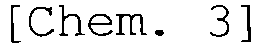

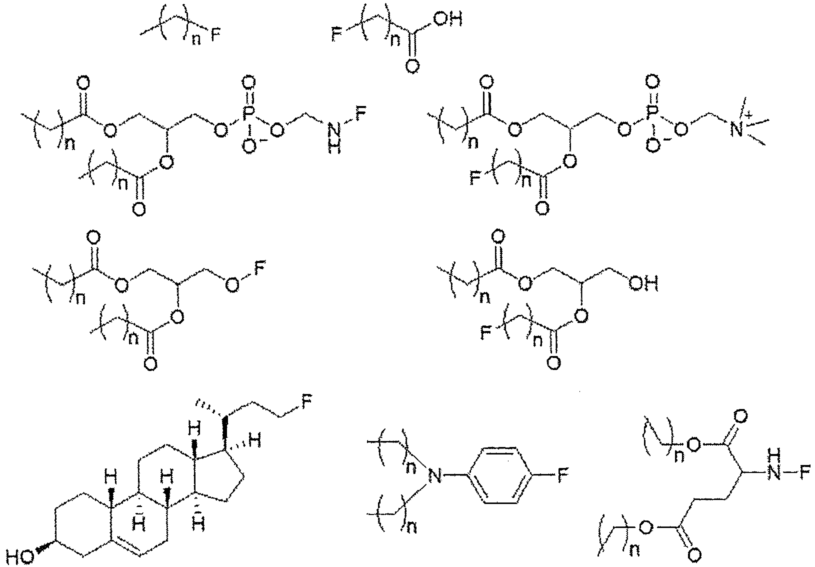

Some biomolecule, such as nicotinamide adenine dinucleotide phosphate, flavins, proteins and amino acids, absorb light having a wavelength of 600 nm or less and emit fluorescence (autofluorescence) having a wavelength of 600 nm or less. For this reason, if a fluorescent probe having a maximum absorption wavelength and a maximum fluorescence emission wavelength each of 600 nm or less is used for detecting a biomolecule, interference due to autofluorescence of contaminant molecules may pose a problem in fluorescence measurement. Therefore, the compound represented by the formula (A) of the present invention is desirably a fluorescent molecule having a maximum absorption wavelength and a maximum fluorescence emission wavelength each of 600 nm or more. In consideration of the action of heat, generated by absorption by water of light having a wavelength of 980 nm or more irradiated on the water, on a temperature-responsive particle, it is more desirably a fluorescent molecule having a maximum absorption wavelength and a maximum fluorescence emission wavelength each of 600 to 900 nm.

In one embodiment, the fluorescent molecule represented by the formula (A) is preferably a squaric acid derivative, a fluorescein derivative, a rhodamine, a coumarin, an oxazine, a pyrene, an anthracene, a phthalocyanine, a cyanine, an azo dye, a carbopyronine, an NBD derivative, a dansyl derivative, a BODIPY fluorophore, an ALEXA fluorophore, a rhodol dye, a naphthalimide, or a porphyrin.

Examples of the aforementioned squaric acid derivative include a compound represented by the following formula I; 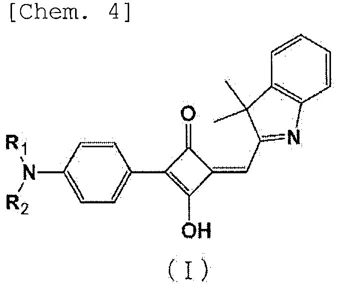 wherein R₁ and R₂ are each independently the same or different hydrophobic group.

The compound represented by the formula (A) or the formula (I) includes not only a free form thereof but also a salt thereof. Examples of such a salt, which varies depending on the type of compound, include a base addition salt such as an inorganic base salt such as alkali metal salt (sodium salt, potassium salt, etc.), alkaline earth metal salt (calcium salt, magnesium salt, etc.), aluminum salt and ammonium salt, and an organic base salt such as trimethylamine, triethylamine, pyridine, picoline, ethanolamine, diethanolamine, triethanolamine, dicyclohexylamine, N,N' - dibenzylethylenediamine, and an acid addition salt such as inorganic acid salt such as hydrochloride, hydrobromide, sulfate, hydroiodide, nitrate and phosphate, and an organic acid salt such as citrate, oxalate, acetate, formate, propionate, benzoate, trifluoroacetate, maleate, tartrate, methanesulfonate, benzenesulfonate and p-toluenesulfonate.

In the aforementioned squaric acid derivative, R₁ and R₂ may be each a straight chain hydrocarbon or a branched-chain hydrocarbon, and may be a saturated hydrocarbon or an unsaturated hydrocarbon. Preferred examples include a straight chain saturated hydrocarbon, but they are not limited thereto as long as the aforementioned fluorescent molecule dissociates in the liquid crystalline phase of the aforementioned molecular assembly and aggregates in the gel phase thereof.

More specific examples of R₁ and R₂ include a hydrocarbon group having 2 to 10 carbon atoms, and more specifically, R₁ and R₂ can be selected from an n-butyl group, an n-pentyl group and an n-hexyl group, but they are not limited thereto as long as dissociation occurs in the liquid crystalline phase of the aforementioned molecular assembly and aggregation occurs in the gel phase thereof.

Fluorescein, carboxyfluorescein, and calcein are known as water-soluble fluorescent molecules that exhibit ACQ (Reference document 1). Fluorescein derivatives incorporating hydrophobic groups include those represented by the formula (2) in which fluorescein is the fluorescent chromophore, and those represented by the formula (3) in which fluorescein isothiocyanate is the fluorescent chromophore. In the formulas (2) and (3), R can be selected from the aforementioned hydrophobic groups, and preferably selected from a straight chain hydrocarbon and diacylphosphatidylethanolamine.

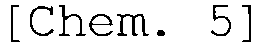

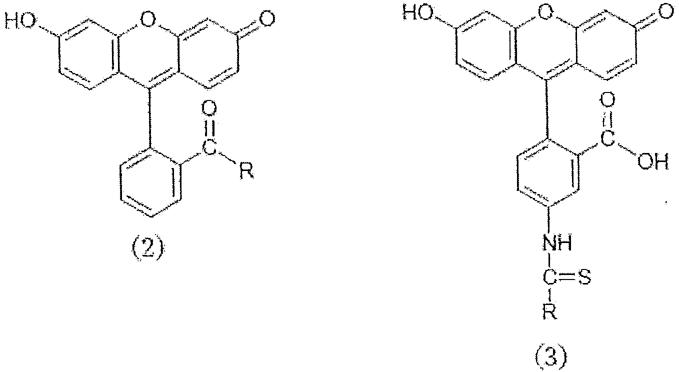

Rhodamines include the formula (4) in which rhodamine B is the chromophore, the formula (5) in which rhodamine B isothiocyanate is the fluorescent chromophore, the formula (6) in which sulforhodamine B is the chromophore, and the formula (7) in which Texas Red is the fluorescent chromophore. In the formulas (4), (5), (6), the ethyl group of rhodamine B may be replaced with a proton to form rhodamine 110, or with a methyl group to form tetramethylrhodamine. In the formulas (4), (5), (6), and (7), R can be selected from the aforementioned hydrophobicity groups, and is preferably straight chain saturated hydrocarbon or diacyl phosphatidylethanolamine. Reference document 4 discloses a method for measuring membrane fusion by utilizing the property of concentration quenching of fluorescent molecules derived from rhodamine B with an octadecyl group, a linear saturated hydrocarbon, introduced thereinto. It has been shown that rhodamines introduced with hydrophobic groups aggregate and quench in lipid bilayer membranes.

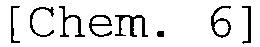

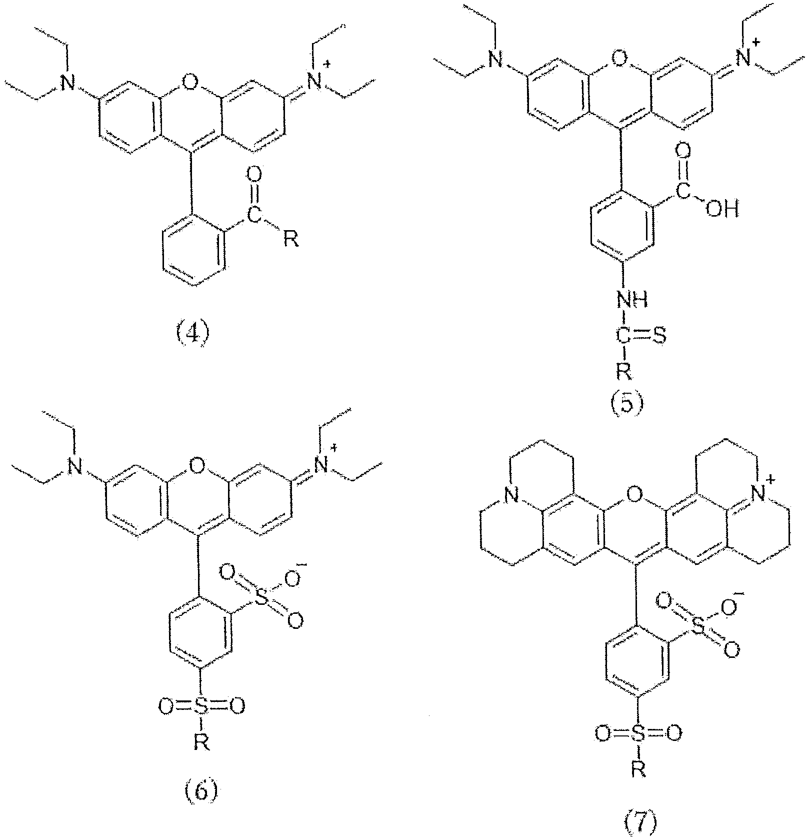

Coumarins include the formula (8) in which coumarin is the fluorescent chromophore. In the formula (8), R can be selected from the aforementioned hydrophobic groups.

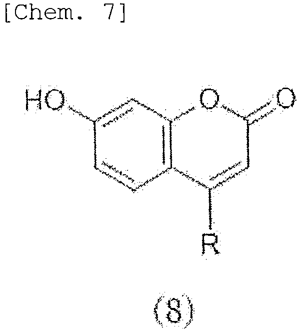

Oxazines include the formula (9) in which resorufin is the fluorescent chromophore, and Nile Red. In the formula (9), R can be selected from the aforementioned hydrophobic groups.

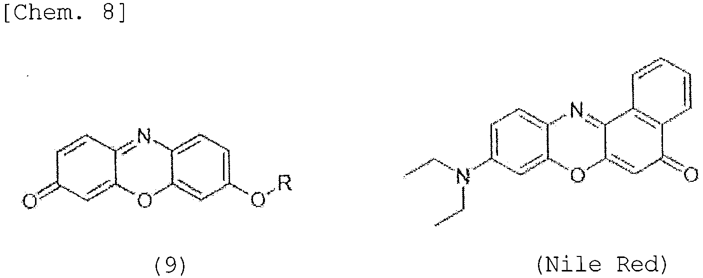

Pyrenes and anthracenes are highly hydrophobic, and they can thus be directly incorporated into the hydrophobic portion of molecular assemblies. Alternatively, they can be chemically incorporated into hydrophobic groups, as shown in the formulas (10) and (11), and R can be selected from the aforementioned hydrophobic groups.

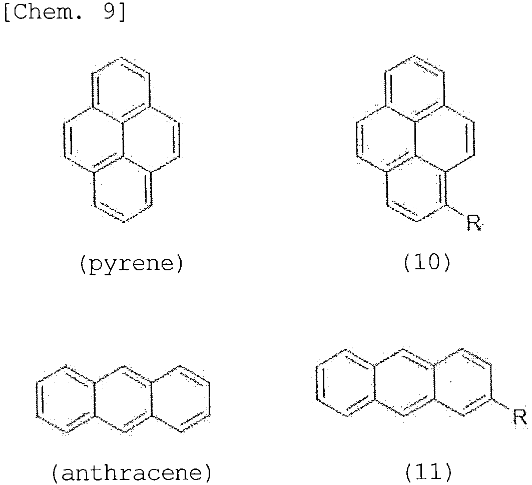

Phthalocyanines are compounds with phthalocyanine as a basic skeleton, and metal complexes with various metals (M) coordinated can be used as fluorescent molecules. Examples of the central metal include iron, cobalt, silicon, zinc, copper, and vanadium. Various phthalocyanine derivatives, in which the protons of the benzyl groups of phthalocyanine are replaced, as well as naphthalocyanine and naphthalocyanine metal complexes can also be used as fluorescent molecules.

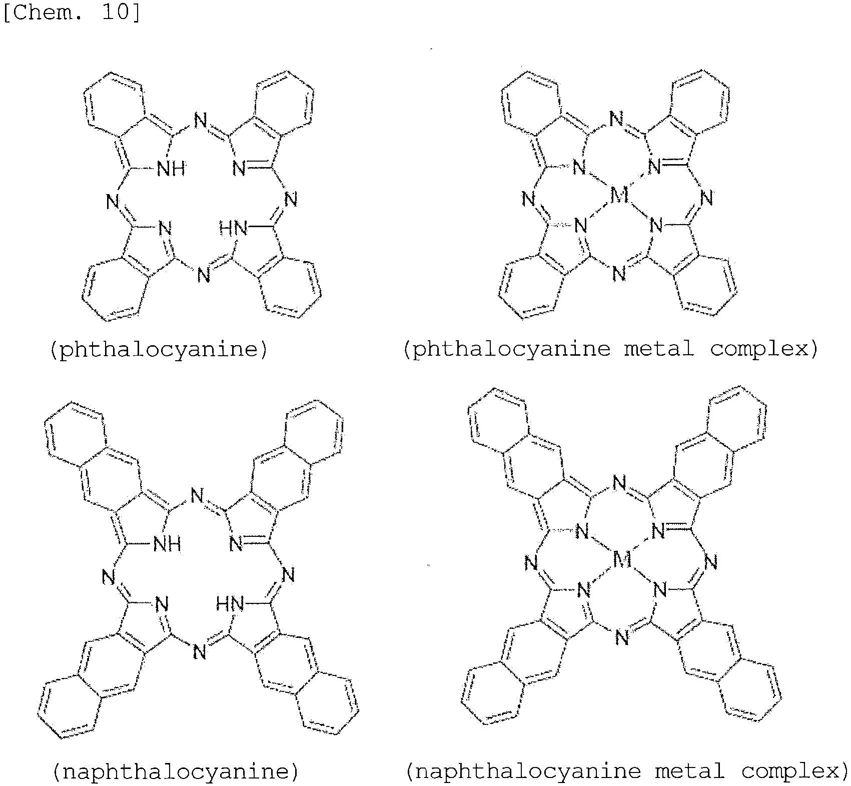

Examples of the cyanine fluorescent molecule include commercially available fat-soluble carbocyanine dyes such as DiIC12(3) (product name), DiD (product name), DiO (product name), DiI (product name), and DiR (product name).

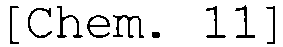

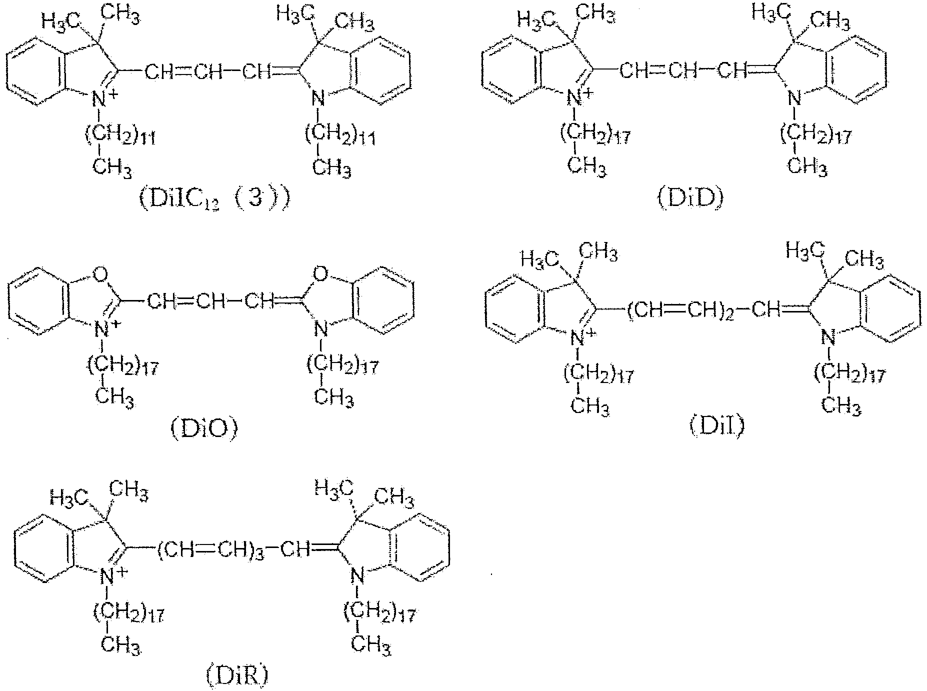

Examples of azo dyes include Sudan III and Oil Red O, which are used to stain neutral fats.

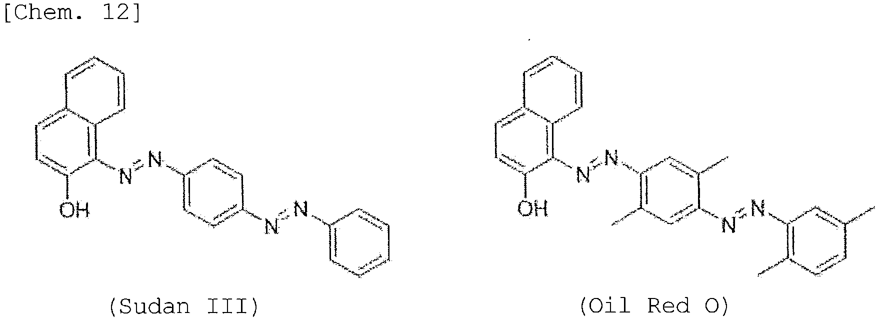

Carbopyronines are fluorescent molecules with the formula (12) as a basic skeleton. In the formula (12), R can be selected from the aforementioned hydrophobic groups. An example of carbopyronine is Atto 633 (product name).

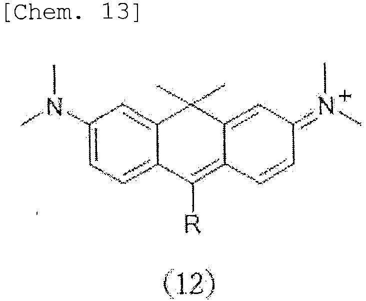

Examples of the NBD derivative include the formula (13), in which NBD is the chromophore. In the formula (13), R can be selected from the aforementioned hydrophobic groups.

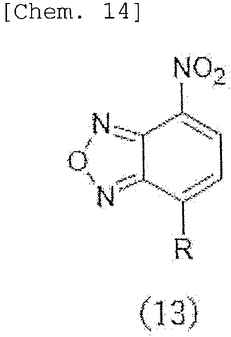

Examples of the dansyl derivative include the formula (14). In the formula (14), R can be selected from the aforementioned hydrophobic groups.

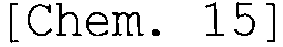

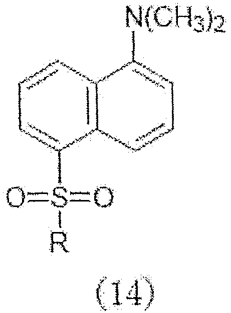

BODIPY fluorophores such as BODIPY 493/503 (product name) and BODIPY 505/515 (product name) can be introduced into the hydrophobic portion of a molecular assembly. They can also be chemically introduced into the hydrophobic group as shown in the formula (15), in which R can be selected from the aforementioned hydrophobic groups.

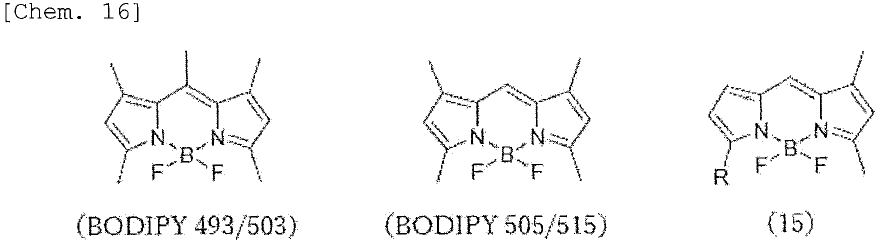

As ALEXA fluorophores, Alexa 350, Alexa 405, Alexa 488, Alexa 532, Alexa 546, Alexa 555, Alexa 561, Alexa 568, Alexa 594, Alexa 647, Alexa 660, Alexa 680, Alexa 700, and Alexa 750 (all product names) are commercially available. These ALEXA fluorophores can be used as amphiphilic fluorescent molecules by conjugating them to hydrophobic groups such as amino group, thiol group, aldehyde ketone, carboxylate or phosphate, azide group, and alkyne group.

Examples of the Rhodol dye include the formulas (16) and (17). In the formulas (16) and (17), R can be selected from the aforementioned hydrophobic groups.

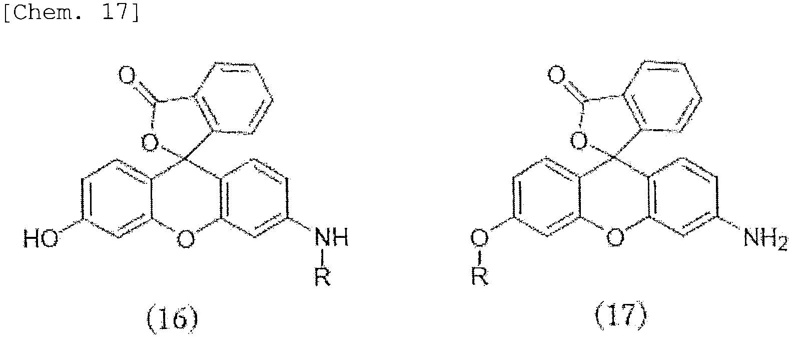

Examples of the naphthalimides include the formula (18). In the formula (18), R can be selected from the aforementioned hydrophobic groups.

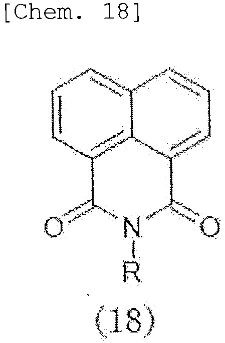

Porphyrins are compounds with porphyrin as the basic skeleton, and metal complexes with various metals (M) coordinated can be used as fluorescent molecules. Examples of the central metal include iron, cobalt, silicon, zinc, copper, and vanadium. They can also be used as amphiphilic molecules in which porphyrin is chemically introduced into the hydrophobic portion.

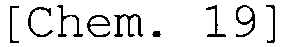

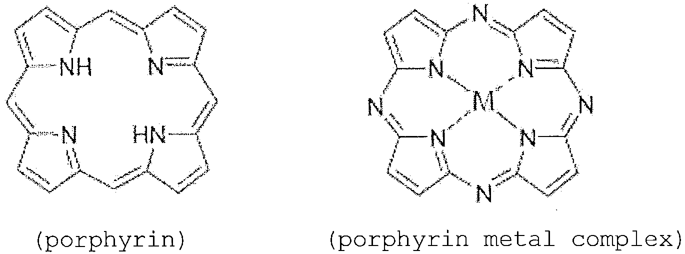

With respect to fluorescent molecules that exhibit ACQ, Reference document 13 describes aggregation-induced emission (AIE) fluorescent molecules that exhibit strong emission property upon aggregation. When using molecular assembly incorporating such AIE fluorescent molecules, it is possible to construct a detection system in which AIE fluorescent molecules in an emitting state upon aggregation in the solid or gel phase are dissociated and quenched upon transition to a liquid phase or liquid crystal due to temperature rise. However, detection systems based on a change from an emitting state to a quenched state (turn off) are known to have lower detection sensitivity than a change from a quenched state to an emitting state (turn on). In addition, because fluorescent molecules generally exhibit a decrease in fluorescence intensity with temperature rise, in detection systems using AIE fluorescent molecules as the fluorescent molecules, the signal and noise exhibit similar fluorescence temperature characteristics and cannot be distinguished. For these reasons, AIE fluorescent molecules are not suitable for use in the present invention. Furthermore, fluorescent molecules whose fluorescence emission property does not change upon aggregation and dissociation cannot be used to construct the detection system of the present technique in which multiple target biomolecules are simultaneously measured using the changes in the fluorescence emission temperature and emission intensity of temperature-responsive fluorescent particles as indicators, and therefore are not encompassed in the present invention.

Examples of the aforementioned amphiphilic molecule include phospholipid.

The type of phospholipid is not particularly limited, but glycerophospholipid (also referred to as diacyl-type phospholipid) is preferable, and in order to form a stable lipid bilayer membrane exhibiting a phase transition, a diacyl-type phospholipid having a phosphocholine group in the hydrophilic portion is particularly preferable. The lengths of acyl chains of diacyl-type phospholipid may be the same as or different from each other. The diacyl-type phospholipid having a phosphocholine group includes saturated phospholipids and unsaturated phospholipids. In the present invention, both of them may be used and they may be used in combination. Examples of the saturated phosphocholine that can be used include synthetic, semisynthetic and natural based phospholipids and derivatives thereof such as hydrogenated egg-yolk lecithin and hydrogenated soybean lecithin with hydrogenation rate close to 100%, and dimyristoyl phosphocholine, dipentadecanoyl phosphocholine, dipalmitoyl phosphocholine, diheptadecanoyl phosphocholine and distearoyl phosphocholine.

Examples of the aforementioned molecular assembly include an emulsion and a lipid nanoparticle having a hydrophobic core surrounded by a monolayer membrane (also referred to as a monolayer), and a vesicle having a closed membrane structure like a spherical shell. Examples of such a vesicle include, but are not limited to, a lipid bilayer membrane vesicle (also referred to as a liposome). The particle that can be used as a temperature-responsive fluorescent particle of the present invention, that is, particle that can be used as the aforementioned molecular assembly is not limited by whether it has a multilayer or monolayer, or particle diameter or the like, as long as it is a molecular assembly that undergoes a solid phase-liquid phase transition or a gel-liquid crystalline phase transition at a specific temperature.

In order to prevent aggregation and fusion of molecular assembly such as lipid vesicle, an amphiphilic molecule having a charged residue or a macromolecular chain in the hydrophilic portion can be included therein as a constituent. The molecular assembly thus obtained such as a lipid vesicle have a high dispersion stability due to electrostatic repulsion by its electric charge or a steric exclusion effect by its macromolecular chain.

Examples of the amphiphilic molecule having a charged residue, when it is a lipid molecule, include phosphatidylglycerol, phosphatidylserine and an anionic phospholipid having phosphatidic acid in the hydrophilic portion, a carboxylic acid-type lipid such as L-glutamic acid, N-(3-carboxy-1-oxopropyl)-, 1,5-dihexadecyl ester (SA), and an amino acid-type lipid having an amino acid in the hydrophilic group.

Examples of the amphiphilic molecule having a macromolecular chain, when it is a lipid molecule, include 1,2-distearoyl-sn-glycero-3-phosphoethanolamine-N-monomethoxy poly(ethylene glycol). Poly(ethylene glycol) is not limited in the molecular weight, but it preferably has a molecular weight of 1000 to 5000 to prevent aggregation of lipid vesicles.

As the amphiphilic molecule, when the molecule is a lipid molecule, 1,2-dimyristoyl-sn-glycero-3-phosphocholine (PC₁₄), 1,2-dipentadecanoyl-sn-glycero-3-phosphocholine (PC₁₅) (manufactured by Avanti Polar Lipids Inc.), 1,2-diheptadecanoyl-sn-glycero-3-phosphocholine (PC₁₇) (manufactured by Avanti Polar Lipids Inc.), 1,2-distearoyl-sn-glycero-3-phosphocholine (PC₁₈), 1,2-dipalmitoyl-sn-glycero-3-phosphocholine (DPPC), 1,2-dioleoyl-sn-glycero-3-phosphocholine (DOPC), 1,5-dihexadecyl-N-succiny-L-glutamate (DHSG), 1,2-distearoyl-sn-glycero-3-phosphoethanolamine (DSPE), and the like can also be used. The gel-liquid crystalline phase transition temperature of the bilayer membrane formed by lipid molecules can be changed by the number of carbon atoms in the acyl chains of the constituent lipid molecules. When simultaneously detecting two or more types of biomolecules, lipid vesicles composed of lipid molecules with different numbers of carbon atoms in the acyl chain can be combined as temperature-responsive fluorescent particles, such as two types of DMPC (phase transition temperature 23°C) and DPPC (phase transition temperature 41°C), two types of PC15 (phase transition temperature 35°C) and PC17 (phase transition temperature 50°C), three types of DMPC (phase transition temperature 23°C) and DPPC (phase transition temperature 41°C), and DSPC (phase transition temperature 55°C), and the like.

Aggregation-caused quenching of fluorescent molecules in a molecular assembly such as lipid vesicles depends on the concentration in the molecular assembly, and the aggregation-caused quenching also decreases with a decrease in the concentration of the fluorescent molecule. Accordingly, the temperature-responsive fluorescent particle of the present invention contains the compound represented by the formula I in a concentration of 0.3 mol % or more, preferably 0.7 mol % or more and more preferably 1.0 mol % or more.

The production of such a temperature-responsive fluorescent particles can be obtained by causing the compound of the formula (I) to coexist during the production of the molecular assembly. As an example of the production of the molecular assembly, it can be produced according to a known method for producing a lipid vesicle (Sheng Dong et al, ACS Appl. Nano Mater. 2018, 1, 1009-1013).

The temperature-responsive fluorescent particles of the present invention obtained as described above are obtained as temperature-responsive fluorescent probe particles by binding of a biomolecular recognition element for detection.

The binding of the temperature-responsive fluorescent particles and a biomolecular recognition element for detection is achieved by a chemical reaction between reactive functional groups on the surface of the temperature-responsive fluorescent particles and reactive functional groups on the biomolecular recognition element for detection. For example, the temperature-responsive fluorescent particles and a biomolecular recognition element for detection are linked by a combination of a maleimide group and a thiol group, an amino group or hydroxyl group, and a carboxyl group, a propargyl group and an azide group, and the like. Alternatively, temperature-responsive fluorescent particles bound to a biomolecular recognition element can be obtained by producing a lipid vesicle modified with a detection antibody according to a known method (WO2000/064413, JP 2003-73258 A, JP 58-134032 A, Manjappa S. A., et al, J. Controlled Release 2011, 150, 2-22, Li T., et al, Nanomed.: Nanotechnol. Biol. Medicine, 2017, 13, 1219-1227).

The magnetic particles bind, via a biomolecular recognition element for capture, to the target biomolecule to be detected or measured, and the below-mentioned temperature-responsive fluorescent particles bind, via a biomolecular recognition element for detection, to the target biomolecule, whereby a complex consisting of the magnetic particles, the target biomolecule, and the below-mentioned temperature-responsive fluorescent particles can be formed. In step (1) of the present invention, the magnetic particles bound to a biomolecular recognition element for capture, biological sample, and temperature-responsive fluorescent probe particles are brought into contact with a magnetic substrate, whereby the temperature-responsive fluorescent particles can be adhered to the magnetic substrate via the formed complex.

For example, when the aforementioned biomolecular recognition element is an antibody, (a) temperature-responsive probe particles composed of temperature-responsive fluorescent particles and a detection antibody, (b) a target biomolecule that is the antigen of the detection antibody and capture antibody, and (c) the capture antibody bound to the magnetic particles form a complex of the temperature-responsive fluorescent probe particles (temperature-responsive fluorescent particles bound to the detection antibody)-target biomolecule (antigen)-capture antibody-magnetic particles, upon which the temperature-responsive fluorescent particle becomes adhered to the magnetic substrate.

The magnetic substrate used in step (1) of the present invention is not particularly limited as long as it is a magnetic substrate, and examples include metal plates (microplates, dishes, and the like). When magnetism is generated at a specific location using a magnet or the like, the substrate does not need to be metal, and insoluble substrates such as polymers (e.g., polystyrene), glass beads, microplates, immunochromatography filter paper, glass filters, and the like can be mentioned. The substrate is preferably a microplate from the viewpoint of ease of handling. The microplate may be provided with respective inlets for introducing magnetic particles bound to a biomolecular recognition element for capture, a biological sample, temperature-responsive fluorescent probe particles, and a buffer solution, a flow channel for forming a complex among the magnetic particles, biological sample, and temperature-responsive fluorescent probe particles, an area for adhering the magnetic particles using a magnet, a detection port for detecting fluorescence, and the like.

The manner in which the magnetic particles bound to a biomolecular recognition element for capture, the biological sample, and the temperature-responsive fluorescent probe particles are brought into contact with the magnetic substrate in step (1) of the present invention is not particularly limited as long as the temperature-responsive fluorescent particles can be adhered to the magnetic substrate via the formed complex. In one embodiment, step (1) of the present invention may contain, for example, the following steps:
(1') a step of bringing a magnetic particle bound to a biomolecular recognition element for capture into contact with a magnetic substrate (step (1') of the present invention),
(2') a step of contacting a biological sample with the aforementioned magnetic substrate (step (2') of the present invention), and
(3') a step of contacting a temperature-responsive fluorescent probe particle with the aforementioned magnetic substrate (step (3') of the present invention).

In step (1') of the present invention, magnetic particles bound to a biomolecular recognition element for capture are brought into contact with a magnetic substrate. By contacting the magnetic particles with the magnetic substrate, the magnetic particles are adhered to the magnetic substrate. Between step (1') and step (2') of the present invention, a step of washing the magnetic substrate may be further included in order to remove unadhered magnetic particles.

In step (2') of the present invention, a biological sample is brought into contact with the magnetic substrate with magnetic particles adhered thereto in step (1') of the present invention. By contacting the biological sample with the magnetic substrate, the target biomolecule in the biological sample is bound to the biomolecular recognition element for capture which has been bound to the magnetic particles, thus resulting in the adhesion of the target biomolecule to the magnetic substrate. Between step (2') and step (3') of the present invention, a step of washing the magnetic substrate may be further included in order to remove unadhered target biomolecule.

In step (3') of the present invention, a temperature-responsive fluorescent probe particle is brought into contact with the magnetic substrate with the target biomolecule adhered thereto in step (2') of the present invention. By contacting the temperature-responsive fluorescent probe particles with the magnetic substrate, the target biomolecule is bound to a biomolecular recognition element for detection which has been bound to the temperature-responsive fluorescent particles, thus resulting in the adhesion of the temperature-responsive fluorescent particle to the magnetic substrate. Between step (3') and step (2) of the present invention, a step of washing the magnetic substrate may be further included in order to remove temperature-responsive fluorescent particles.

In another embodiment, step (1) of the present invention may contain, for example, the following steps:
(1") a step of bringing a magnetic particle bound to a biomolecular recognition element for capture into contact with a biological sample,
(2") a step of contacting a complex obtained in step (1") with a magnetic substrate,
(3") a step of contacting a temperature-responsive fluorescent probe particle with the aforementioned complex, and
(4") a step of contacting the complex obtained in step (3") with the magnetic substrate.

In step (1") of the present invention, a biological sample is brought into contact with a magnetic particle bound to a biomolecular recognition element for capture. By contacting the biological sample with the magnetic particles, the target biomolecule in the biological sample is bound to the biomolecular recognition element for capture which has been bound to the magnetic particles, thus resulting in the formation of a complex consisting of the magnetic particle bound to the biomolecular recognition element for capture and the target biomolecule.

In step (2") of the present invention, the complex obtained in step (1") is brought into contact with the magnetic substrate. By contacting the complex obtained in step (1") with the magnetic substrate, the magnetic particle adheres to the magnetic substrate, thus resulting in the adhesion of the target biomolecule to the magnetic substrate. Between step (2") and step (3") of the present invention, a step of washing the magnetic substrate may be further included in order to remove complexes, magnetic particles, and target biomolecules that were not adhered.

In step (3") of the present invention, a temperature-responsive fluorescent probe particle is brought into contact with the complex of the target biomolecule and magnetic particle in step (2'') of the present invention. At this time, the complex may be left adhered to the magnetic substrate, but the contact area increases by removing the magnetic field and mixing and contacting the temperature-responsive fluorescent probe particles with the complex, which in turn promotes binding between the biomolecular recognition element for detection, which has been bound to the temperature-responsive fluorescent particles, and the target biomolecule, and a complex of the temperature-responsive fluorescent particle and the magnetic particle is formed via the target biomolecule. In step (4") of the present invention, by contacting the complex obtained in step (3") with the magnetic substrate, the temperature-responsive fluorescent particle is adhered to the magnetic substrate. Between step (4") and step (2) of the present invention, a step of washing the magnetic substrate may be further included in order to remove unadhered temperature-responsive fluorescent particles.

In still another embodiment, step (1) of the present invention may contain, for example, the following steps:
(1‴) a step of contacting a temperature-responsive fluorescent probe particle with a biological sample,
(2‴) a step of contacting a complex obtained in step (1‴) with a magnetic particle bound to a biomolecular recognition element for capture, and
(3‴) a step of contacting the complex obtained in step (2‴) with a magnetic substrate.

In step (1‴) of the present invention, a biological sample is brought into contact with a temperature-responsive fluorescent probe particle. By contacting the biological sample with the temperature-responsive fluorescent probe particle, the target biomolecule in the biological sample is bound to the temperature-responsive fluorescent probe particle, thus resulting in the formation of a complex consisting of the temperature-responsive fluorescent probe particle and the target biomolecule.

In step (2‴) of the present invention, the complex obtained in step (1‴) is brought into contact with a magnetic particle bound to a biomolecular recognition element for capture. By contacting the complex obtained in step (1‴) with the magnetic particles bound to the biomolecular recognition element for capture, the target biomolecule in the biological sample is bound to the biomolecular recognition element for capture, which has been bound to the magnetic particle, thus resulting in the formation of a complex consisting of the magnetic particle bound to the biomolecular recognition element for capture, the target biomolecule, and the temperature-responsive fluorescent probe particle.

In step (3‴) of the present invention, the complex obtained in step (2‴) is brought into contact with a magnetic substrate. By contacting the complex obtained in step (2‴) with the magnetic substrate, the magnetic particle in the aforementioned complex adheres to the magnetic substrate, thus resulting in the adhesion of the aforementioned complex to the magnetic substrate.

The concentration of the magnetic particles used in step (1), step (1'), step (1"), or step (2‴) of the present invention is not particularly limited. The magnetic particles are preferably contained in the solution at a concentration of 5 µg/ml or more, more preferably 10 µg/mL or more, further preferably 100 µg/mL or more, particularly preferably 500 µg/mL or more, at the time of contact. The upper limit value is not particularly limited and is preferably 10 mg/mL or less, more preferably 5 mg/mL or less, further preferably 1 mg/mL or less.

The concentration of the temperature-responsive fluorescent probe particles used in step (3'), step (3"), or step (1‴) of the present invention is not particularly limited. The fluorescent molecule contained in the probe particles is preferably contained in the solution at a concentration of 0.55 µg/mL or more, more preferably 1.0 µg/mL or more, further preferably 5.0 µg/mL or more, at the time of contact. The upper limit value is not particularly limited and is preferably 50 µg/mL or less, more preferably 40 µg/mL or less, further preferably 30 µg/mL or less.

The magnetic particles bound to a biomolecular recognition element for capture, biological sample, temperature-responsive fluorescent probe particles, or a complex formed therefrom may be in a dispersed state in an aqueous solvent at the time of contact with the magnetic substrate. The aqueous solvent is not particularly limited and, for example, a buffer can be used. Examples of the buffer include an acetate buffer, a phosphate buffer, a citrate buffer, a citrate-phosphate buffer, a borate buffer, a tartrate buffer, a Tris buffer, phosphate-buffered saline (PBS), McIlvaine buffer, solutions (e.g., PBS-T, etc.) containing these and a nonionic surfactant (e.g., Tween 20, etc.), and the like.

The conditions for the above-mentioned each contact are not particularly limited, and the contacting is generally performed at a temperature of 0 to 45°C, preferably at a temperature of 0 to 40°C, more preferably at a temperature of 4 to 37°C, further preferably at a temperature of 25°C to 37°C. The contact time is also not particularly limited, and it is typically 6 hr or less, preferably 2 hr or less, more preferably 1 hr or less. The lower limit of the contact time is also not particularly limited, and it is typically 10 sec or more, preferably 1 min or more, more preferably 10 min or more.

A step of washing the aforementioned magnetic substrate may be further included between step (1) and step (2), step (1') and step (2'), step (2') and step (3'), step (3') and step (2), step (2'') and step (3"), step (4") and step (2), and step (3‴) and step (2) of the present invention. By washing, magnetic particles, biological samples, temperature-responsive fluorescent probe particles, and complexes formed therefrom that were not adhered onto the magnetic substrate can be removed. The solvent to be used for washing is not particularly limited and, for example, an aqueous solvent is used. The aqueous solvent is not particularly limited and, for example, a buffer can be used. Examples of the buffer include an acetate buffer, a phosphate buffer, a citrate buffer, a citrate-phosphate buffer, a borate buffer, a tartrate buffer, a Tris buffer, phosphate-buffered saline (PBS), McIlvaine buffer, solutions (e.g., PBS-T, etc.) containing these and a nonionic surfactant (e.g., Tween 20, etc.), and the like.

As described in the below-mentioned Examples, magnetic particles with hydrophilic surfaces to which streptavidin was immobilized and multiple temperature-responsive fluorescent particles with avidin on the surface and having different lipid acyl chain lengths (temperature-responsive fluorescent probe particles that emit fluorescence at mutually different temperatures) were brought into contact with a magnetic substrate, the substrate was heated, and change in fluorescence intensity was observed over time. As a result, it was confirmed that the fluorescence intensity changes over time depending on the temperature at which the thermal transition of the temperature-responsive fluorescent particles occurs (i.e., transition from solid to liquid phase or transition from gel phase to liquid crystalline phase). From the above, the present inventors have found that two or more types of biomolecules can be detected or quantified simultaneously in the method for detecting or quantifying biomolecules of the present invention, by using two or more types of magnetic particles bound to mutually different biomolecular recognition elements for capture, and two or more types of temperature-responsive fluorescent particles bound to mutually different biomolecular recognition elements for detection and having different lipid acyl chain lengths. Therefore, step (1) of the present invention may include the following characteristics:
(i) the biomolecules to be detected or quantified are two or more types
(ii) the magnetic particles are two or more types of magnetic particles bound to mutually different biomolecular recognition elements for capture
(iii) the temperature-responsive fluorescent probe particles are two or more types of temperature-responsive fluorescent particles which are bound to mutually different biomolecular recognition elements for detection and have different lipid acyl chain lengths.

In step (2) of the present invention, the magnetic substrate to which the temperature-responsive fluorescent particles have been adhered in step (1), step (3'), step (4"), or step (3‴) is heated.

When the magnetic substrate is heated, the temperature of the aforementioned complex rapidly increases due to heat conduction from the magnetic substrate. In this case, the temperature-responsive fluorescent particles in the complex undergo a phase transition from the gel phase to the liquid crystalline phase, so that the fluorescent molecules in the temperature-responsive fluorescent particles that aggregated and were quenched in the gel phase dissociate and emit fluorescence. On the other hand, when the target biomolecule, that is, antigen, is not present in a biological sample, the aforementioned complex is not formed. Therefore, the temperature-responsive fluorescent particles cannot adhere to the magnetic substrate and remain suspended in the solution or have been removed by washing. As a result, the quenched state is maintained even when the magnetic substrate is heated.

In step (2) of the present invention, the method for heating the magnetic substrate includes, but is not limited to, a method of placing the substrate on a thermoplate or hot plate or in a water bath, that has been set to a desired temperature. When using such an apparatus, the set temperature and heating time of the apparatus can be determined appropriately by those skilled in the art depending on the temperature at which the temperature-responsive fluorescent particles undergo a phase transition from gel phase to liquid crystalline phase. Furthermore, when the magnetic substrate is not made of metal and the temperature-responsive fluorescent particles are adhered to the substrate using a magnet, it is sufficient to heat only the adhered portion.

In step (3) of the present invention, the fluorescent molecules in the temperature-responsive fluorescent particles heated in step (2) dissociate, and the emitted fluorescence is measured.

In step (3) of the present invention, the method for measuring fluorescence emission is not particularly limited, and fluorescence emission can be measured by using a general optical fiber-type fluorescence detector, a microplate reader, a fluorescence microscope and a detector equipped with a camera (such as a CCD camera) (such as Celvin). Further, as in the process in the ELISA method, the concentration of the target biomolecule in a biological sample can be quantitatively determined by using a calibration curve generated using a sample having a known concentration of the target biomolecule (reference standard).

An example of a reactor for continuously performing steps (1) to (3) of the present invention is a microfluidic chip. The microfluidic chip to be used preferably has inlets for the sample, detection reagent, and washing solution, and a structure including a magnetic particle washing tank, a stirring/mixing tank, and a detection tank, which are arranged in this order along the direction of liquid flow in the flow channel. Fluorescence is measured while heating the magnetic particles collected in the detection tank using a magnetic substrate.

The present invention also provides a kit for detecting or quantifying a biomolecule in a biological sample (hereinafter, the kit for detecting or quantifying biomolecule of the present invention).

The kit for detecting or quantifying biomolecule of the present invention includes (1) a magnetic particle bound to a biomolecular recognition element for capture, and (2) a temperature-responsive fluorescent probe particle which is a temperature-responsive fluorescent particle bound to a biomolecular recognition element for detection, and can further include a microfluidic chip as a detection device. The (1) magnetic particle bound to a biomolecular recognition element for capture, and (2) temperature-responsive fluorescent probe particle which is a temperature-responsive fluorescent particle bound to a biomolecular recognition element for detection may be the same as those used in the method for detecting or quantifying a biomolecule of the present invention. The kit of the present invention may contain other reagents and the like in addition to the above-mentioned (1) - (2), and these reagents and the like may be previously combined with the above-mentioned (1) - (2) or may be stored in separate containers. Examples of the reagent and the like include an aqueous solvent, a reference standard having a known concentration of biomolecules, a positive control, a negative control, instructions describing protocols, and the like. These reagents and the like can be mixed in advance if necessary.

All literatures referred to in the present specification are incorporated herein by reference in their entirety. Examples described herein illustrate embodiments of the present invention and should not be construed as limiting the scope of the present invention.

### [Example]

### (Example 1) Synthesis of fluorescent dye (SQR22)

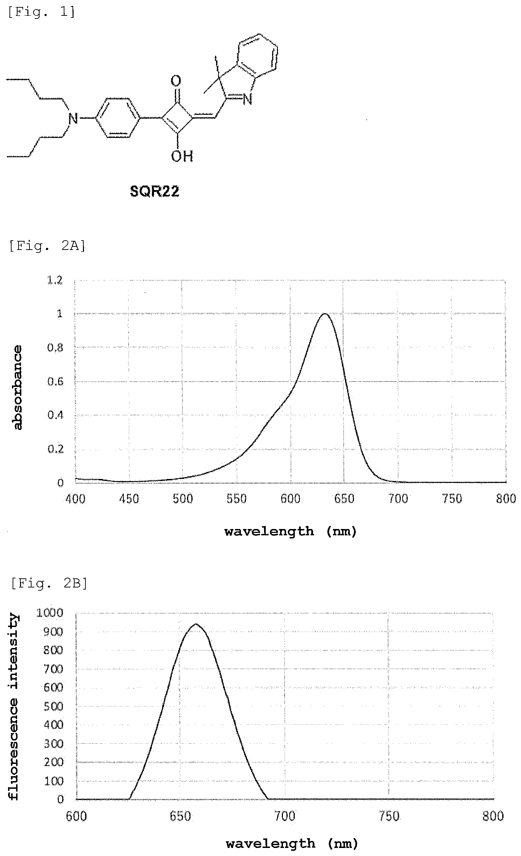

Squaric fluorescent dye (SQR22) was synthesized according to the method of a previous report (Sou K. et al., Scientific Reports, 2019, 9, 17991). The structure of SQR22 was identified by NMR and mass spectrometry. The structural formula of SQR22 is shown in Fig. 1.
¹H NMR (CDCl₃, 400 MHz, δ ppm): 0.89 (t, J = 7.3 Hz, 6H, -CH₃); 1.25-1.35 (m, 4H, -CH₂-); 1.41 (s, 6H, -CH₃); 1.50-1.57 (m, 4H, -CH₂-); 3.27-3.31 (m, 4H, -CH₂-); 5.75 (s, 1H, =CH-); 6.60 (d, J = 9.2 Hz, 2H, ArH), 7.04-7.36 (m, 4H, ArH); 8.03 (d, J = 9.1 Hz, 2H, ArH). ¹³C NMR (CDCl₃, 400 MHz, δ ppm): 14.12, 20.47, 25.64, 29.61, 50.12, 51.07, 89.87, 111.95, 113.27, 118.85, 122.74, 125.18, 128.82, 130.95, 140.06, 141.59, 151.19, 169.22, 181.33, 181.80, 185.51, 187.28. MS (ESI) m/z (M⁺) 443.

### (Example 2) Synthesis of fluorescent dye (SQR23)

The fluorescent dye (SQR23) was synthesized by modifying a previously reported method (Dong, S. ACS Appl. Nano Mater. 2018, 1, 1009-1013). The synthesis scheme of SQR23 is shown in scheme 1.

First, precursors N,N-dibutylaniline 1 and squaryl chloride 2 were synthesized as previously reported (Li F., et al. Chem. Eur. J. 20, 9991-9997 (2014); Easwaran A., et al. J. Am. Chem. Soc. 2004, 126, 6590-6598). The 1 and 2 were reacted with each other under reflux of toluene for 6 hr to obtain a chloride intermediate. This chloride intermediate was hydrolyzed in a mixed solvent of 2N hydrochloric acid and acetic acid/water (1/1) to obtain compound 3. Compound 3 and 2,3,3-trimethylindolenine 4 were reacted under reflux of n-butanol/toluene (4:1 mixed solution). The reaction product was separated using a silica gel column (mobile phase: hexane/ethyl acetate (2:1)) to obtain SQR23. The structure of SQR23 was identified by ¹H NMR and mass spectrometry.
¹H NMR (CDCl₃, 400 MHz, δ ppm): 0.90 (t, 6H, -CH₃); 1.25-1.35 (m, 16H, -CH₂-); 1.50 (s, 6H, -CH₃); 1.60-1.70 (m, 4H, -CH₂-); 3.27-3.31 (m, 4H, -CH₂-); 5.85 (s,1H, =CH-); 6.70 (d, 2H, ArH), 7.18-7.40 (m, 4H, ArH); 8.10 (d, 2H, ArH). MS m/z[M-H]⁻ 525. 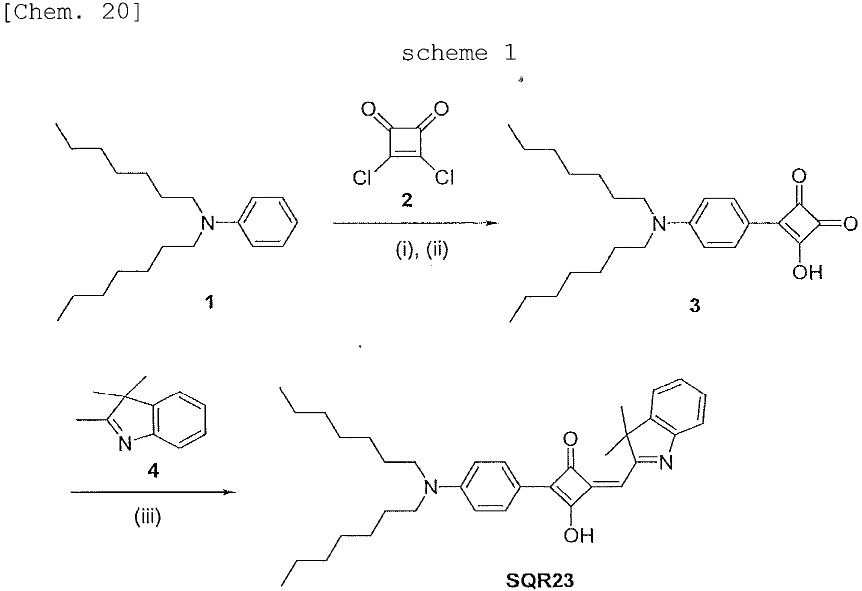
(i) toluene, reflux, 6 hr
(ii) 2N hydrochloric acid, acetic acid/water (1:1), reflux, 2 hr
(iii) n-butyl alcohol/toluene (4:1), reflux, 3 hr

### (Example 3) Spectroscopic property of SQR23

SQR23 was dissolved in ethanol, and the ultraviolet visible absorption spectrum (Fig. 2A) and fluorescence emission spectrum (Fig. 2B) were measured using an ultraviolet visible spectrophotometer (V-670, manufactured by JASCO Corporation) and a fluorescence spectrophotometer (RF-5300PC, manufactured by Shimadzu Corporation). In ethanol, the maximum absorption wavelength of SQR23 was 633 nm, and the maximum fluorescence emission wavelength was 658 nm.

### (Example 4) Introduction of SQR dye into liposomes with different phase transition temperatures

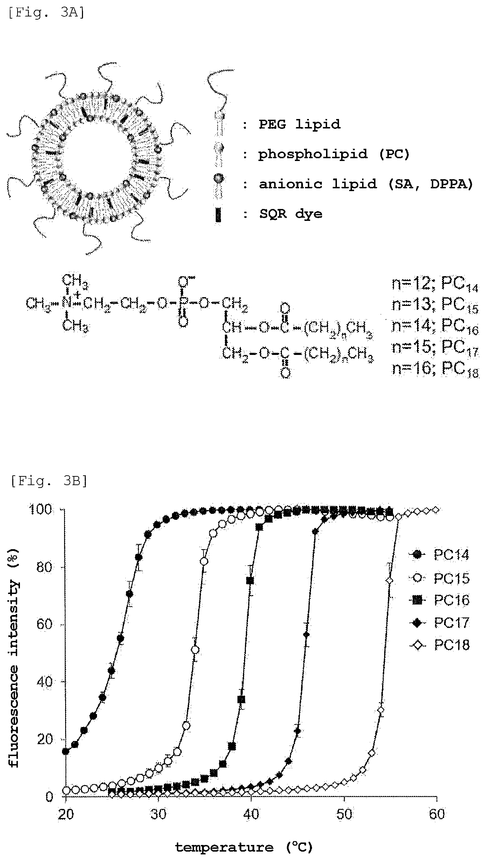

Three types of amphiphilic molecules: 1,2-diacyl-sn-glycero-3-phosphocholine (PC), L-glutamic acid, N-(3-carboxy-1-oxopropyl)-, 1,5-dihexadecyl ester (SA) (manufactured by Nippon Fine Chemical Co., Ltd.), and 1,2-distearoyl-sn-glycero-3-phosphoethanolamine-N-monomethoxy poly(ethylene glycol) (5000) (PEG-DSPE) (manufactured by NOF CORPORATION) were dissolved in t-butanol at a molar ratio of PC/SA/PEG-DSPE=9/1/0.06 and lyophilized to obtain a mixed lipid powder. Five types of PC were used, each with different acyl chain lengths: 1,2-dimyristoyl-sn-glycero-3-phosphocholine (PC14 or DMPC) (manufactured by Avanti Polar Lipids Inc.), 1,2-dipentadecanoyl-sn-glycero-3-phosphocholine (PC15) (manufactured by Avanti Polar Lipids Inc.), 1,2-dipalmitoyl-sn-glycero-3-phosphocholine (PC16 or DPPC) (manufactured by Nippon Fine Chemical Co., Ltd.), 1,2-diheptadecanoyl-sn-glycero-3-phosphocholine (PC17) (manufactured by Avanti Polar Lipids Inc.), and 1,2-distearoyl-sn-glycero-3-phosphocholine (PC18) (manufactured by Avanti Polar Lipids Inc.) (Fig. 3A). To the obtained mixed lipid powder was added a stock solution of SQR22 (in ethanol) (at a weight ratio of the mixed lipid/SQR22=50/1), the resultant was dissolved in t-butanol and lyophilized to obtain an SQR22-containing mixed lipid powder. The SQR22-containing mixed lipid powder was dispersed in phosphate-buffered saline (PBS) (5 mg/mL), and hydrated and dispersed with a vortex mixer. This dispersion was added to EXTRUDER (manufactured by Northern Lipids Inc.), and caused to successively permeate through polycarbonate membrane filters (manufactured by Merck Millipore) having pore sizes of 0.45 µm, 0.20 µm, and 0.05 µm at 60°C under pressure to obtain an SQR22-containing liposome dispersion (lipid vesicle) dispersion (FIG. 2A). As shown in Fig. 3A, the amphiphilic SQR dye is introduced into the lipid bilayer membrane that forms the liposome.

### (Example 5) Fluorescence emission property of SQR22-containing liposomes

The correlation curve between the fluorescence intensity and temperature of SQR22-containing liposome is shown in Fig. 3B. The fluorescence intensity of SQR22 introduced into a lipid bilayer membrane changes in different temperature ranges depending on the lipid species constituting the bilayer membrane. The gel-liquid crystalline phase transition temperatures of lipid bilayer membranes composed of PC14, PC15, PC16, PC17, and PC18 are 24, 35, 40, 49, and 55°C, respectively, and the temperature range in which the fluorescence intensity of SQR22 changes rapidly is nearly identical to the phase transition temperature of the lipid bilayer membrane. This is because SQR22 aggregates and is in a quenched state when the lipid bilayer membrane is in the gel phase, but SQR22 aggregation is dissociated and transition to an emitting state occurs when the lipid membrane is heated above the phase transition temperature and undergoes transition to the liquid crystalline phase. By using SQR-containing temperature-responsive liposomes (TLips), it is possible to prepare a series of temperature-responsive fluorescent particles with different fluorescence emission temperatures depending on the gel-liquid crystalline phase transition temperature of the lipid bilayer membrane.

### (Example 6) Preparation of biotin-modified temperature-responsive liposomes

Three types of lipid molecules: 1,2-dipalmitoyl-sn-glycero-3-phosphocholine (DPPC) (manufactured by Nippon Fine Chemicals Co., Ltd.), 1,2-dipalmitoyl-sn-glycero-3-phosphatidic acid (DPPA) (manufactured by NOF Corporation), and 1,2-distearoyl-sn-glycero-3-phosphoethanolamine-N-[biotinyl(polyethylene glycol)-2000] (ammonium salt) (B-PEG-DSPE) (manufactured by Avanti Polar Lipids), and SQR23 were dissolved in cyclohexane at DPPC/DPPA/B-PEG-DSPE/SQR23 = 86.6/9.6/0.5/3.3 (molar ratio) and lyophilized to obtain a mixed lipid powder containing SQR23. The obtained SQR22-containing mixed lipid powder was dispersed in 0.02% sodium azide phosphate-buffered saline (PBS) (4 mg/mL), and the mixed lipid powder was hydrated and dispersed by stirring the dispersion for 40 min while heating to 50°C. This dispersion was added to EXTRUDER (manufactured by Northern Lipids Inc.), and passed through a polycarbonate membrane filter (manufactured by Whatman) having a pore size of 0.20 µm twice at 58°C under pressure to obtain a dispersion of temperature-responsive liposome (B-TLip) carrying biotin on the surface.

### (Example 7) Temperature-responsive fluorescence emission property of B-TLip

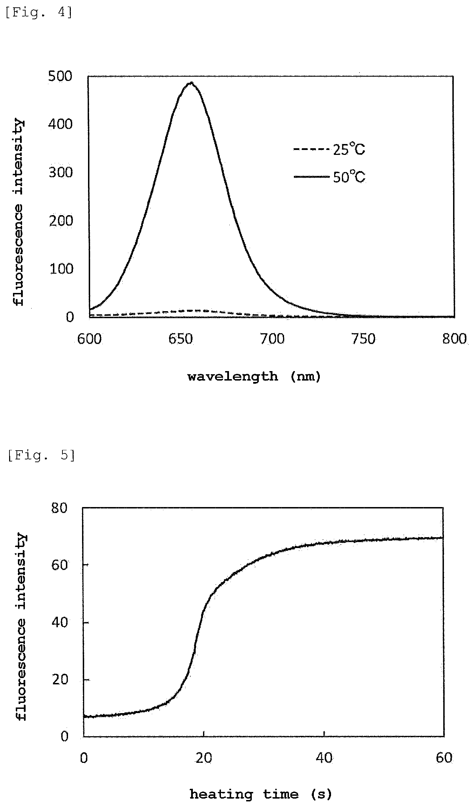

The fluorescence emission spectrum (excitation wavelength = 570 nm) of the B-TLip dispersion ([SQR23] = 1 µg/mL) was measured using a fluorescence spectrophotometer (RF-5300PC, manufactured by Shimadzu Corporation). To confirm the temperature-responsive fluorescence emission property, the measured was performed at a dispersion temperature of 25°C, and then the temperature of the dispersion was raised to 50°C and the measurement was performed. As shown in Fig. 4, SQR23 was quenched at 25°C, but when heated to 50°C which is above the phase transition temperature of the DPPC bilayer membrane (41°C), strong fluorescence emission was observed. When the DPPC bilayer membrane is in the gel phase, SQR23 aggregates and is in a quenched state; however, when the lipid membrane is heated above the phase transition temperature and transition to a liquid crystalline phase occurs, aggregation of SQR23 dissociates and transition to an emissive state occurs. SQR23-containing liposomes exhibit temperature-responsive fluorescence properties similar to those of the SQR22-containing liposomes shown in Example 5.

Next, a B-TLip dispersion ([SQR23] = 1 µg/mL, 60 µL) was injected into a flat-bottom 1 x 8 strip well (manufactured by Thermo Fisher Scientific), and the strip was placed on a glass plate at 80°C and heated. The change in fluorescence value over time is shown in Fig. 5. The profile in which the fluorescence intensity increases rapidly and reaches a constant value as the temperature of the dispersion increases due to heating from the bottom of the strip.

### (Example 8) Detection of binding via biotin-streptavidin molecular recognition by B-TLip and streptavidin-modified magnetic particles

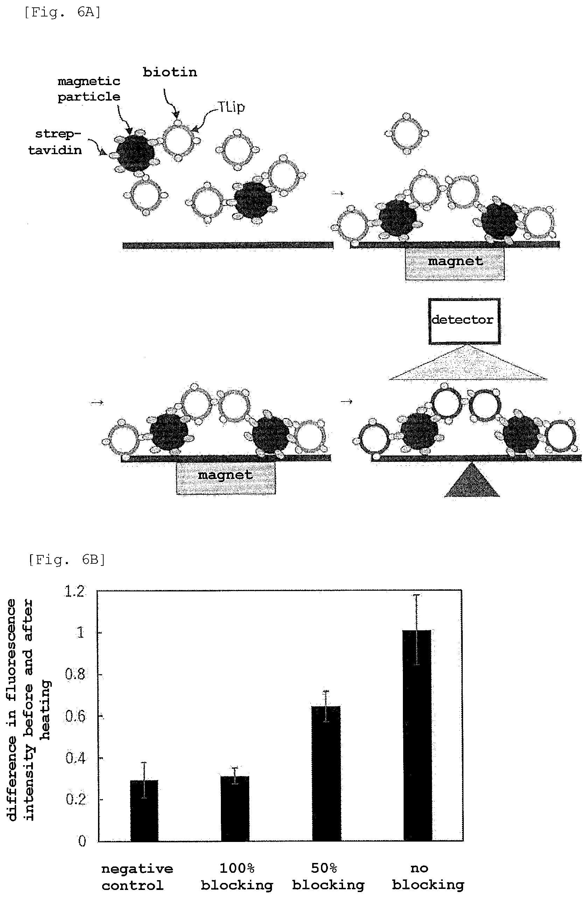

To a flat-bottom 1 x 8 strip well were added 60 µL of magnetic particles with hydrophilic surfaces to which streptavidin was immobilized (Magnosphere^{™} MS300/Streptavidin (manufactured by Medical and Biological Laboratories)) to afford a final streptavidin concentration of 0.50 µg/mL and 60 µL of B-TLip to afford a final SQR23 concentration of 1 µg/mL, and the well was left standing at room temperature for 10 min. After the reaction, the magnetic particles were pulled to the bottom of the well with a magnet, and the well was washed twice with 200 µL of DPBS and B-TLip that was not bound to the magnetic particles was removed. Then, while heating the flat-bottom 1 x 8 strip well on a hot plate heated to 80°C, the change in fluorescence intensity over time was measured using an optical fiber probe type fluorescence detector (FLE 1100, manufactured by Nippon Sheet Glass Co., Ltd.). A schematic diagram of the detection system is shown in Fig. 6A. Magnetic particles without immobilized streptavidin (Magnosphere^{™} MS300/Carboxyl) (no streptavidin modification) and a sample with [streptavidin]=50 µg/mL were compared, using magnetic particles with streptavidin blocked by adding an excess amount of biotin (100% blocking) as a negative control (n=3).

As shown in Fig. 6B, the fluorescence intensity of the negative control (no streptavidin modification) and one in which streptavidin was 100% blocked with an excess amount of biotin showed similar values. Therefrom it was considered that the blocking of streptavidin on the magnetic particles and B-TLip unbound to the magnetic particles were removed by washing. When the streptavidin blocking rate was reduced to 50%, the difference in fluorescence intensity increased, and without blocking, the difference in fluorescence intensity increased further. Therefrom it was considered that the binding of TLip to magnetic particles through molecular recognition of biotin and streptavidin can be detected by the increase in fluorescence intensity due to heating, and that the extent of the increase in TLip fluorescence intensity reflects the amount of TLip bound to the magnetic particles.

### (Example 9) Preparation of liposome for SARS-CoV-2 detection

Three types of lipid molecules: 1,2-dipalmitoyl-sn-glycero-3-phosphocholine (DPPC) (manufactured by Nippon Fine Chemicals Co., Ltd.), 1,2-dipalmitoyl-sn-glycero-3-phosphatidic acid (DPPA) (manufactured by NOF Corporation), and N-[(3-Maleimide-1-oxopropyl)aminopropyl polyethyleneglycol-carbamyl]distearoylphosphatidyl-ethanolamine (MAL-PEG-DSPE, SUNBRIGHT DSPE-020MA) (manufactured by NOF CORPORATION) and SQR23 were dissolved in cyclohexane at DPPC/DPPA/B-PEG-DSPE/SQR23 = 86.6/9.6/0.5/3.3 (molar ratio) and lyophilized to obtain a mixed lipid powder containing SQR23. The obtained SQR22-containing mixed lipid powder was dispersed in 0.02% sodium azide phosphate-buffered saline (PBS) (4 mg/mL), and the mixed lipid powder was hydrated and dispersed by stirring the dispersion for 40 min while heating to 50°C. This dispersion was added to EXTRUDER (product name, manufactured by Northern Lipids Inc.), and passed through a polycarbonate membrane filter (manufactured by Whatman) having a pore size of 0.20 µm twice at 58°C under pressure to obtain a dispersion of SQR23-containing temperature-responsive liposome (Mal-TLip) carrying a maleimide group on the surface.

Next, in order to bind a detection monoclonal antibody (IgG-9) that specifically recognizes the SARS-CoV-2 nucleocapsid protein to Mal-TLip, sulfhydryl groups were introduced into the IgG by using 2-iminothiolane hydrochloride (Traut's reagent). 2-Iminothiolane hydrochloride was dissolved in Trout's solution (25 mM HEPES, 140 mM NaCl, 3 mM EDTA) and added to the IgG solution (IgG/2-iminothiolane hydrochloride = 1/20 (molar ratio)), and the mixture was shaken at room temperature for 1 hr to allow the reaction to proceed. In order to remove unreacted 2-iminothiolane hydrochloride, the reaction solution was passed through a gel filtration chromatography column (PD-10 column, manufactured by Cytiva), and the fraction containing IgG with a sulfhydryl group (IgG-SH) introduced thereinto was collected. Since the obtained IgG-SH solution was diluted, it was concentrated using a centrifugal ultrafiltration filter (50 kDa Amicon Ultrafilter, manufactured by Millipore) and used in the next reaction.

To the Mal-TLip dispersion (SQR23 concentration: 80 µg/mL) was added 0.2-fold volume of IgG-SH solution (IgG-SH concentration: 0.7 mg/mL), and the mixture was allowed to react overnight at room temperature with gentle shaking on a shaker. Thereafter, 0.8 mM N-acetyl-L-cysteine solution was added to the reaction mixture, and the mixture was shaken at room temperature for 10 min to block unreacted maleimide groups. The reaction mixture was passed through a chromatography column (mobile phase: PBS containing 0.02% sodium azide) packed with a gel filtration carrier (Sephacryl s-300 HR, manufactured by GE Healthcare), and the liposome fraction showing the blue color of SQR23 was collected. IgG-SH and N-acetyl-L-cysteine that did not bind to Mal-TLip could be separated because they were eluted in a fraction different from the liposome fraction showing the blue color of SQR23.

### (Example 10) Analysis of antibody-labeled temperature-responsive liposomes containing SQR23

The particle size distribution and zeta potential of the recovered liposome fractions were measured using dynamic light scattering and electrophoretic light scattering (Zeta-Sizer Nano ZS, Malvern Panalytical Ltd.). The SQR23 concentration in the dispersion was determined from the absorbance (631 nm) of SQR23 in ethanol. The DPPC concentration was measured using a commercially available phospholipid measurement kit (Phospholipid C-Test Wako, FUJIFILM Wako Pure Chemical Corporation) based on the choline oxidase-DAOS method. The IgG concentration was measured using fluorescamine, which reacts with the amino group of IgG to emit fluorescence. As shown in Table 1, the liposome particle size was controlled to about 200 nm, confirming that temperature-responsive liposomes carrying the detection monoclonal antibody IgG-9 (IgG-TLip) were obtained.

**[Table 1]**

| Table 1. Property of antibody-labeled temperature-responsive liposomes (IgG-TLip) | |
|---|---|
| particle size distribution (nm) | 185±52 |
| zeta potential (mV) | -8.0±0.9 |
| SQR23 concentration (µg/mL) | 17.8 |
| DPPC concentration (µg/mL) | 905.6 |
| IgG concentration (µg/mL) | 25.6 |
| dispersion medium | 0.02% sodium azide-containing PBS |

### (Example 11) Preparation of magnetic particles labeled with capture antibody

Following the product protocol, a capture antibody was immobilized on magnetic particles coated with a hydrophilic polymer and introduced with carboxyl groups as reactive functional groups (Magnosphere^{™} MS300/Carboxyl (particle diameter: 3 µm). First, a Magnosphere^{™} MS300/Carboxyl dispersion (particle content: 1%) was stirred for 10 seconds using a vortex mixer to uniformly disperse the particles. Then, 1 mL of the dispersion (equivalent to 10 mg of particles) was then dispensed into a reaction microtube. The magnetic particles were captured using a magnetic stand and the supernatant was removed. 1 mL of 0.1 M 2-(N-morpholino)ethanesulfonic acid (MES) buffer (pH 5.0) was then added as a reaction buffer, and the mixture was stirred for 10 seconds using a vortex mixer to disperse the magnetic particles. Then, 100 µL of the condensation agent: 1-ethyl-3-[3-dimethylaminopropyl]carbodiimide hydrochloride, dissolved in 0.1 M MES buffer at 10 mg/mL, was added, and the mixture was allowed to react at room temperature for 30 min while admixing in a shaker. Furthermore, 100 µg of a monoclonal antibody (IgG-7) for capture that specifically binds to the SARS-CoV-2 nucleocapsid protein was added to this reaction solution, and the mixture was stirred for 10 seconds in a vortex mixer, after which the mixture was allowed to react at room temperature for 3 hr by inversion admixing. After completion of the reaction, the magnetic particles were captured using a magnetic stand. The supernatant was removed using a Pipetman. 1 mL of washing PBS-T (10 mM PO₄³⁻, pH 7.4, 0.14 M NaCl, 2.7 mM KCl, 0.05% Tween 20) was added and the particles were dispersed by stirring in a vortex mixer for 10 seconds. This washing cycle was repeated three times in total. Finally, the magnetic particles labeled with the monoclonal antibody IgG-7 for capture were redispersed in 1 mL of PBS-T and stored in a refrigerator at 4°C until use.

### (Example 12) Detection of SARS-CoV-2 recombinant N antigen using capture antibody-labeled magnetic particles IgG-TLip

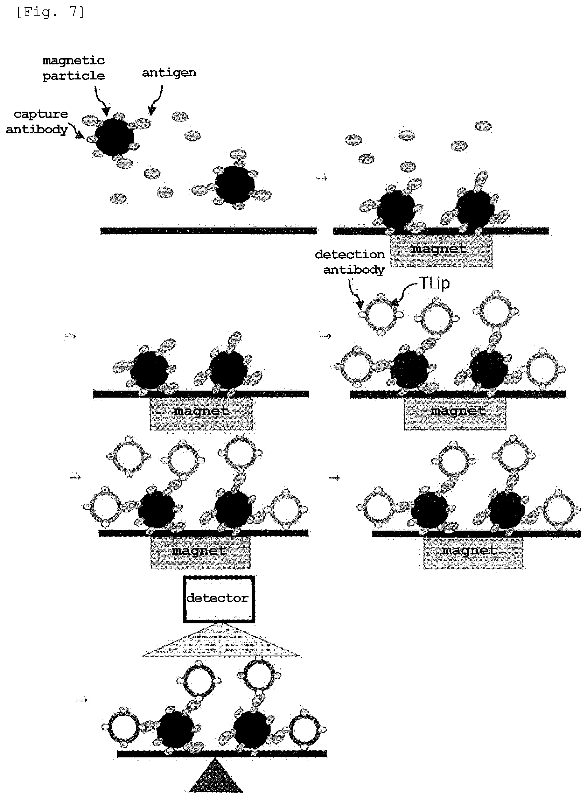

A schematic diagram of antigen detection using magnetic particles and IgG-TLip in a microwell is shown in Fig. 7. Magnetic particles labeled with the capture antibody IgG-7 were diluted to a solid concentration of 1 mg/mL and 60 µL was added to a flat-bottom 1 x 8 strip well. Next, 60 µL of SARS-CoV-2 recombinant N antigen was added to achieve final concentrations of 0, 10, 100 pg/mL, 1, and 100 ng/mL, and they were incubated at room temperature for 0.5, 1, 2.5, 5, and 10 min. The beads were attracted using a magnet, and unbound antigen was removed by washing with 200 µL of PBS. 60 µL of IgG-TLip was added to achieve a final concentration of [SQR23] = 0.5 µg/mL, and they were left standing for the same time as the recombinant N antigen reaction to allow the IgG-TLip to bind to the antigen. Similarly, using a magnet, unbound IgG-TLip was removed with 200 µL of PBS, and the fluorescence intensity over time was measured using an optical fiber probe fluorescence detector (FLE 1100, manufactured by Nippon Sheet Glass Co., Ltd.) while heating on a hot plate (n=3).

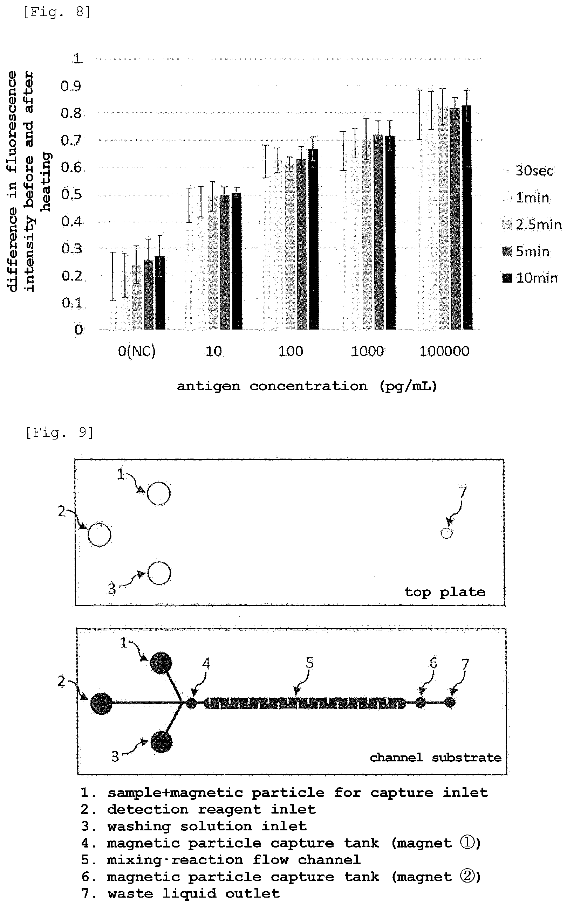

The difference in fluorescence intensity before and after heating, for the incubation time and antigen concentration for the antigen-antibody reaction, and the standard deviation thereof are shown in Fig. 8. From the results, it is clear that the difference in fluorescence intensity increases as the antigen concentration in the sample increases, but that there is no significant change in the difference in fluorescence intensity between reaction times from 30 seconds to 10 min when compared at the same antigen concentration. Therefore, it is considered that the capture of antigen by magnetic particles and the binding of IgG-TLip to the captured antigen proceed rapidly, reaching equilibrium after 30 seconds of incubation.

### (Example 13) Creation of microfluidic channels

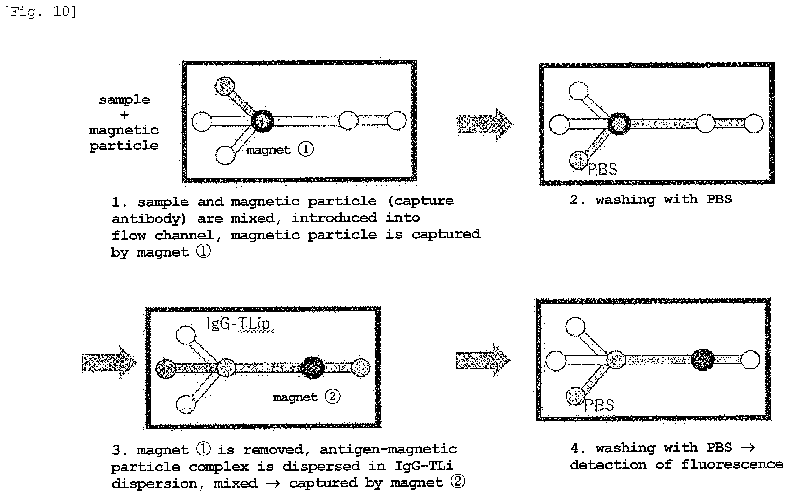

In order to create microfluidic channels, acrylic plates were cut and excavated using a laser processing machine. Holes were drilled in the upper acrylic plate (top plate) according to the design shown in Fig. 9, and holes were excavated in the lower acrylic plate (flow channel substrate) according to the design shown. After processing the acrylic plates, acrylic cutting dust generated by the laser cutter was removed using ultrasonic cleaning, and the plates were dried under reduced pressure. The two acrylic plates, the top plate and the flow channel substrate, were then welded together with an adhesive to create the flow channel. The two acrylic plates were left pressed together in a vice and allowed to dry and harden overnight. Fig. 10 is a schematic diagram showing the injection and flow of solutions into a microfluidic device.

### (Example 14) Detection of antigen using microfluidic channel

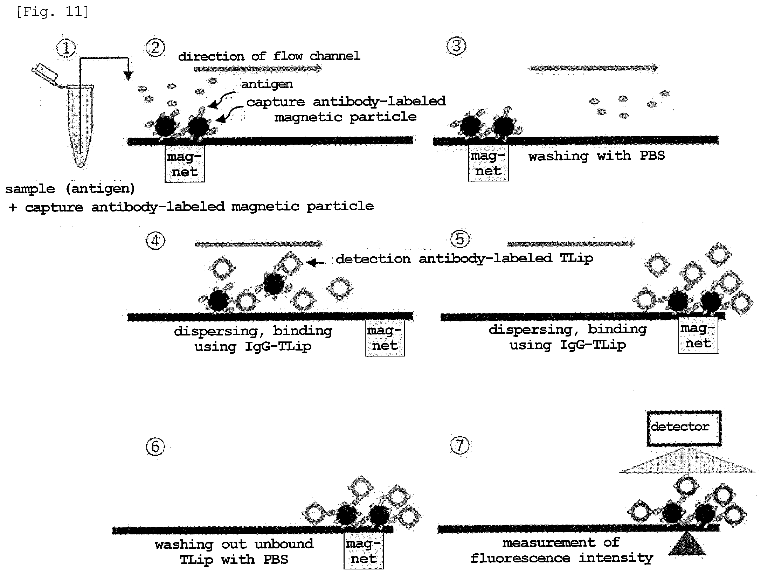

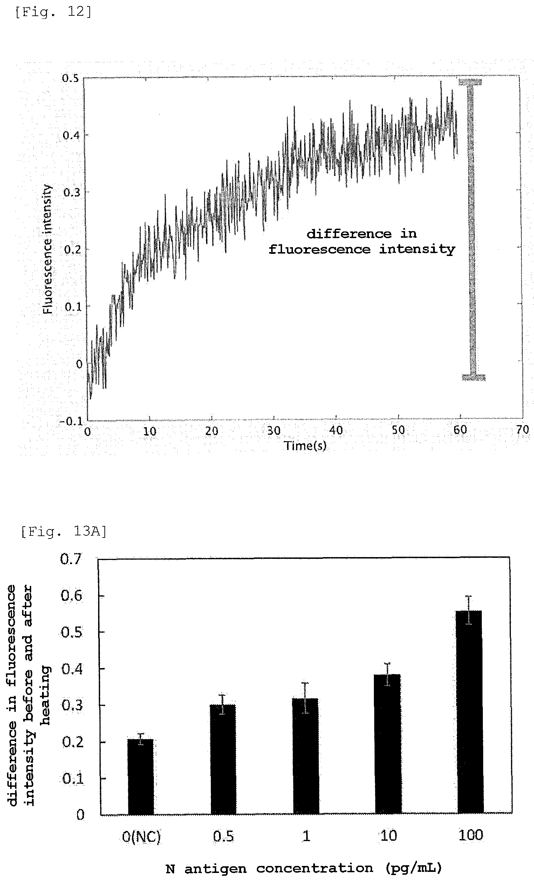

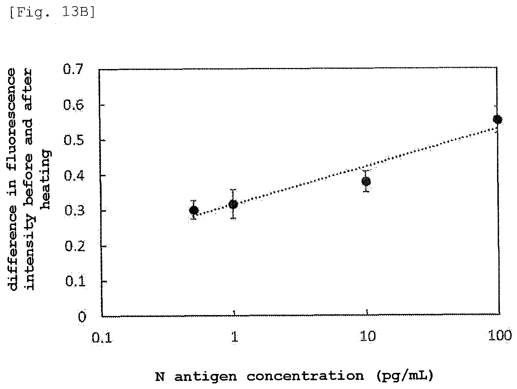

The process for antigen detection using a microfluidic channel created using IgG-T Lip and capture antibody-labeled magnetic particles is shown in Fig. 11. Detection of SARS-CoV-2 recombinant N antigen was performed according to this schematic diagram. First, a syringe pump was connected to the flow channel using a pipette port · tube. 60 µL of sample solution (SARS-CoV-2 recombinant N antigen) was mixed in a 1.5 mL tube with 60 µL of capture antibody-labeled magnetic particle dispersion diluted to a solid concentration of 1 mg/mL, and the mixture was then introduced into the flow channel using a syringe pump. After capturing the magnetic particles with a magnet, PBS (400 µL, 400 µL/min) was flowed through the flow channel to remove unbound recombinant N antigen. Then, the magnet capturing the magnetic particles was removed, and IgG-TLip ([SQR23] = 1 µg/mL, 100 µL) was flowed through the flow channel at 400 µL/min, and the magnetic particles were captured by the magnet in the detection section. Then, PBS (400 µL) was flowed through the flow channel at 400 µL/min to wash out the unbound IgG-TLip, and fluorescence was detected using an optical fiber probe fluorescence detector (FLE 1100, manufactured by Nippon Sheet Glass Co., Ltd.) while heating the detection section (n=3). As shown in Fig. 12, the fluorescence intensity increased with heating, and the difference in fluorescence intensity before and after heating was analyzed. As shown in Fig. 13A and Fig. 13B, the difference in fluorescence intensity increased as the N antigen concentration increased, and a good proportional relationship was observed between the logarithm of the N antigen concentration and the difference in fluorescence intensity. Based on the standard deviation (σ) of the negative control, the detection limit (3σ) was calculated to be 0.21 pg/mL, and the quantification limit (10σ) was calculated to be 2.34 pg/mL.

By using magnetic particles and a microfluidic device, the detection sensitivity of TLip-LISA for SARS-CoV-2 was improved and quantitative. Furthermore, by using magnetic particles to promote the antigen-antibody reaction, the total time until detection was shortened to 10 min. This detection method enables highly sensitive quantitative antigen testing in a test time comparable to that of commercially available qualitative antigen test kits based on immunochromatography (time required for determination: about 15 min).

### (Example 15) Simultaneous detection of heterologous antigens using different response temperatures of TLip

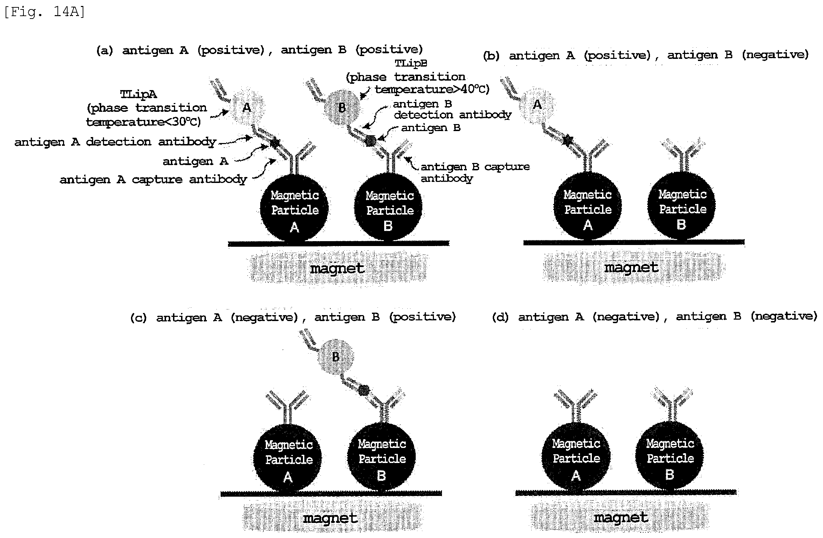

As shown in Example 5, by incorporating SQR dye into liposomes that exhibit a gel-liquid crystalline phase transition, a series of temperature-responsive fluorescent particles can be prepared that exhibit fluorescence emission dependent on the phase transition temperature of the lipid bilayer membrane. Utilizing this property, a method for detecting heterologous antigens in a single measurement was invented. A schematic diagram explaining this method is shown in Fig. 14A. TLipA (phase transition temperature < 30°C) is labeled with detection antibody A, which recognizes antigen A, and magnetic particle A is labeled with capture antibody A, which recognizes antigen A. Therefore, when antigen A is present in the sample, TLipA and magnetic particle A are bound via antigen A. On the other hand, TLipB (phase transition temperature> 40°C) is labeled with detection antibody B, which recognizes antigen B, and magnetic particle B is labeled with capture antibody B, which recognizes antigen B. If antigen B is present in the sample, TLipB and magnetic particle B are bound via antigen B. Fig. 14A(a) shows the case when both antigen A and antigen B are present in the sample, and TLipA and TLipB, which have bound to the magnetic particles, can be collected at the detection site by a magnet. Fig. 14A(b) shows the case when antigen A is present but antigen B is not in the sample. Since detection antibody B and capture antibody B do not react with antigen A, only TLipA binds to the magnetic particles. Conversely, Fig. 14A(c) shows the case when antigen A is not present in the sample but antigen B is. Since detection antibody A and capture antibody A do not react with antigen B, only TLipB binds to the magnetic particles. If neither antigen A nor antigen B is present in the sample, as shown in Fig. 14A(d), neither TLipA nor TLipB binds to the magnetic particles.

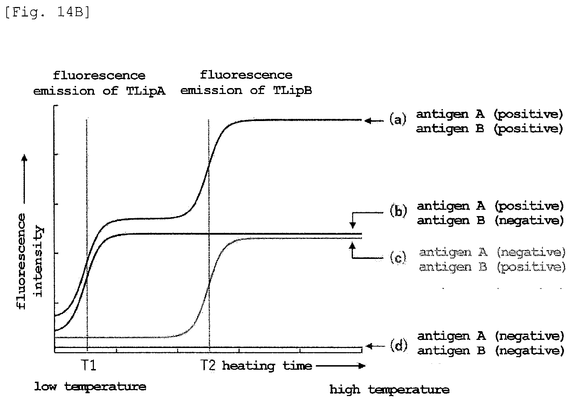

Therefore, by collecting magnetic particles at the detection site by using a magnet and obtaining the information that (a) both TLipA and TLipB are detected, (b) only TLipA is detected, (c) only TLipB is detected, or (d) neither TLipA nor TLipB is detected, the presence or absence of antigen A and antigen B can be simultaneously determined. The fluorescence emission temperature of TLips varies depending on the phase transition temperature of the liposomes. For example, in TLipA labeled with a detection antibody for antigen A, the main component is DMPC having a phase transition temperature of 23°C, while in TLipB labeled with a detection antibody for antigen B, the main component can be DPPC having a phase transition temperature of 41°C. Since TLipA and TLipB have different emission temperatures, the time until fluorescence emission differs when fluorescence intensity is measured while heating. As shown in Fig. 14B, the fluorescence emission at time T1 corresponds to that of TLipA, and the fluorescence emission at T2 corresponds to that of TLipB. By analyzing this fluorescence emission time, the presence or absence of antigen A and antigen B can be detected in a single measurement.

### (Example 16) Differences in emission time due to difference in TLip response temperature

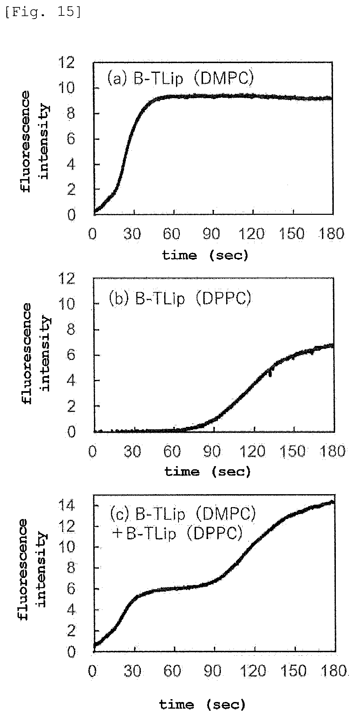

To more specifically demonstrate the method described in Example 15, two types of TLip with different phase transition temperatures were prepared. B-TLip (DPPC) composed of DPPC, DPPA, B-PEG-DSPE, and SQR22 was prepared according to the method described in Example 7. B-TLip (DMPC) composed of DMPC, DPPA, B-PEG-DSPE, and SQR22 was prepared by a similar method. The obtained B-TLip dispersion ([SQR22]=2.5 µg/mL, 120 µL) was injected into a flat bottom 1 x 8 strip well, and while heating the strip well from room temperature (20°C) on a hot plate heated to 65°C, the change in fluorescence intensity over time was measured using an optical fiber probe type fluorescence detector (FLE 1100, manufactured by Nippon Sheet Glass Co., Ltd.). As shown in Fig. 15, the fluorescence intensity of B-TLip (DMPC) increased immediately after the start of heating (Fig. 15a), whereas the fluorescence intensity of B-TLip (DPPC) increased 80 seconds after the start of heating (Fig. 15b). This indicates a clear difference in the time from the start of heating to light emission between B-TLip (DMPC) and B-TLip (DPPC). This difference in the time up to light emission is due to the fact that the gel-liquid crystalline phase transition temperature of B-TLip composed of DMPC is 23°C, whereas that of B-TLip composed of DPPC is 41°C. Furthermore, in a mixture ([SQR22] = 5 µg/mL, 120 µL) of B-TLip (DMPC) ([SQR22] = 5 µg/mL, 60 µL) and B-TLip (DPPC) ([SQR22] = 5 µg/mL, 60 µL), an increase in fluorescence intensity due to B-TLip (DMPC) was observed immediately after the start of heating, and then an increase in fluorescence intensity due to B-TLip (DPPC) was observed from after around 80 seconds (Fig. 15c). Therefore, even when B-TLips composed of DMPC or DPPC with different phase transition temperatures are mixed, each can be detected in a single measurement due to the difference in emission time.

### (Example 17) Simultaneous detection of multiple targets using TLip fluorescence emission time

Using two types of B-TLip with different phase transition temperatures, as described in Example 16, the simultaneous detection method illustrated in Example 15 was examined using the fluorescence emission of B-TLips composed of DMPC or DPPC bound to streptavidin-modified magnetic particles via biotin-streptavidin molecular recognition.

According to the method described in Example 8, to a flat-bottom 1 x 8 strip well were added 60 µL of magnetic particles with hydrophilic surfaces to which streptavidin was immobilized (Magnosphere^{™} MS300/Streptavidin (manufactured by Medical and Biological Laboratories)) to afford a final streptavidin concentration of 0.50 µg/mL and 60 µL of B-TLip (DMPC) or B-TLip (DPPC) to afford a final SQR22 concentration of 1 µg/mL, and the well was left standing at room temperature for 10 min. Then, (a) B-TLip (DMPC) reaction mixture 60 µL + untreated magnetic particles 60 µL, (b) B-TLip (DPPC) reaction mixture 60 µL + untreated magnetic particles 60 µL, (c) B-TLip (DMPC) reaction mixture 60 µL + B-TLip (DPPC) reaction mixture 60 µL were added to a 384-well plate, the magnetic particles were pulled to the bottom of the well with a magnet, the well was washed twice with 100 µL of DPBS, and B-TLip that was not bound to the magnetic particles was removed. Then, the magnetic particles were dispersed in DPBS (40 µL) and, while heating the 384-well plate bottom surface on a hot plate heated to 65°C, the change in fluorescence intensity over time was measured using an optical fiber probe type fluorescence detector (FLE 1100, manufactured by Nippon Sheet Glass Co., Ltd.).

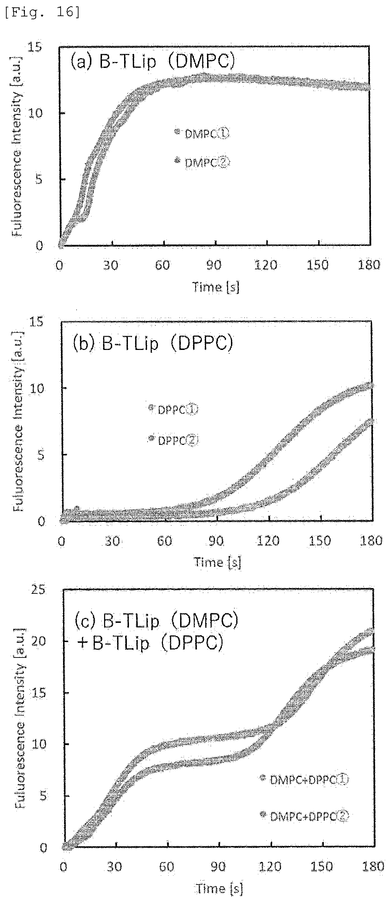

As shown in Fig. 16, the fluorescence intensity of B-TLip (DMPC) which was bound to magnetic particles via biotin-streptavidin molecular recognition increased immediately after the start of heating (Fig. 16a), whereas the fluorescence intensity of B-TLip (DPPC) increased from 90 seconds later (Fig. 16b), and a clear difference in the time from the start of heating to light emission was found even between B-TLip (DMPC) and B-TLip (DPPC) bound to magnetic particles. Furthermore, even in a system composed of a mixture of B-TLip (DMPC) and TLip (DPPC) bound to magnetic particles, a clear difference in the emission time was confirmed between B-TLip (DMPC) and TLip (DPPC) (Fig. 16c).

The method illustrated in Example 9 allows labeling of TLip with any detection antibody, and the method illustrated in Example 11 allows labeling of magnetic particles with any capture antibody. Therefore, for example, two detection antibodies for two different antigens (A and B) are bound to the surfaces of two TLips (A and B) with different phase transition temperatures, respectively. Furthermore, two capture antibodies for two different antigens are bound to the surfaces of two magnetic particles, respectively. It was shown that by simultaneously using the obtained two TLips and two magnetic particles, different target antigens can be simultaneously detected from the analysis of the fluorescence emission times, as illustrated in Figs. 14A and 14B. As illustrated in Fig. 3B in Example 5, temperature-responsive liposomes that emit light at various temperatures can be prepared, and with the common fluorescent dye, a detector equipped with one light source and one light receiver can simultaneously detect various antigens.

### (Example 18) Preparation of liposome reagents and magnetic particles for influenza A detection

Three types of lipid molecules (DMPC, DPPA, MAL-PEG-DSPE, and SQR23) and SQR23 were dissolved in cyclohexane in a molar ratio of DMPC/DPPA/MAL-PEG-DSPE/SQR23 = 86.6/9.6/0.5/3.3 and lyophilized to obtain a mixed lipid powder containing SQR23. According to the method described in Example 9, a liposome reagent carrying a monoclonal antibody for detection (HL1103, GeneTex) that binds to influenza A nucleocapsid was prepared. In addition, according to the method described in Example 11, magnetic particles carrying a monoclonal antibody for capture (HL1089, GeneTex) that binds to influenza A nucleocapsid were also prepared.

### (Example 19) Detection of Influenza A N antigen (nucleocapsid protein)

Using the influenza A detection TLip (DMPC) prepared in Example 18 and magnetic particles, recombinant influenza A N antigen was detected. 30 µL of magnetic particles labeled with antibody for capture (solid concentration: 10 µg/mL) and 30 µL of TLip dispersion carrying antibody for detection ([SQR23] = 20 µg/mL) were mixed in a flat-bottom 384-well plate. Recombinant influenza A N antigen (origin: Puerto Rico/8/1934 H1N1) was added to this mixture to a final concentration of 50-400 ng/mL and allowed to stand at room temperature for 5 min. The magnetic particles were attracted using a magnet, the solution was removed, and the plate was washed with PBS. The magnetic particles were then dispersed in 30 µL of PBS. The fluorescence intensity over time was measured using an optical fiber probe fluorescence detector (FLE 1100, manufactured by Nippon Sheet Glass Co., Ltd.) while heating the bottom surface of the well plate on a hot plate (70°C).

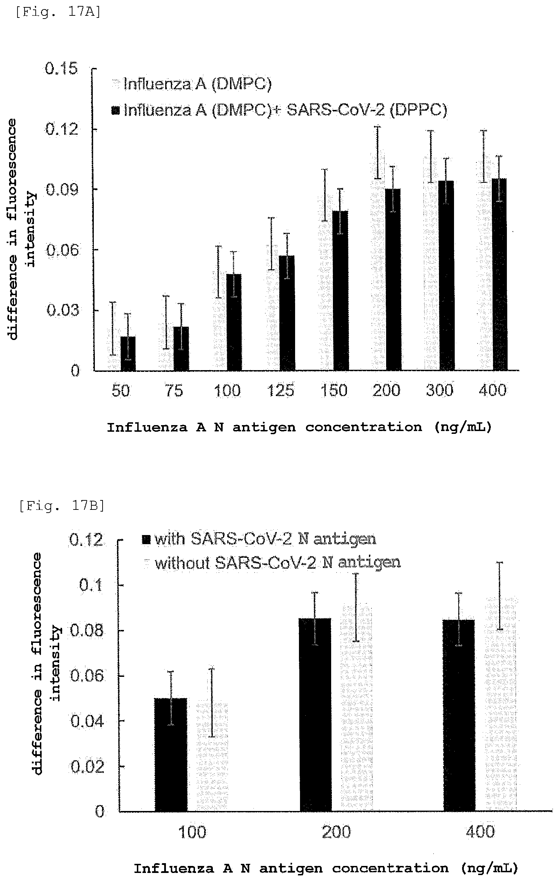

As shown in Fig. 17A, since the difference in fluorescence intensity before and after heating increased with increasing influenza A N antigen concentrations, it was shown that the antigen concentration can be quantitatively detected. The co-presence of TLip carrying SARS-CoV-2 antibody (DPPC) did not affect the difference in fluorescence intensity, which confirms that the co-presence of TLip (DPPC) for SARS-CoV-2 detection minimally affects the detection of influenza A antigen by TLip (DPPC) for influenza A detection. Also, as shown in Fig. 17B, it was confirmed that the co-presence of SARS-CoV-2 N antigen did not affect the detection of influenza A antigen by influenza A detection TLip (DMPC).

### (Example 20) Detection of SARS-CoV-2 and Influenza A N antigen (nucleocapsid protein)

To a flat bottom microwell were added 60 µL of magnetic particles labeled with SARS-CoV-2 capture antibody (solid content concentration: 1 mg/mL) and 60 µL of magnetic particles labeled with influenza A capture antibody (solid content concentration : 1 mg/mL). To this mixture were added 1. Influenza A recombinant N antigen (origin: Puerto Rico/8/1934 H1N1) (1 µg/mL, 60 µL) + DPBS (60 µL), 2. SARS-CoV-2 recombinant N antigen (200 ng/mL, 60 µL) + DPBS (60 µL), 3. Influenza A recombinant N antigen (origin: Puerto Rico/8/1934 H1N1) (1 µg/mL, 60 µL) + SARS-CoV-2 recombinant N antigen (200 ng/mL, 60 µL), and 4. DPBS (200 µL), and the mixture was allowed to stand at room temperature for 2 min to capture the antigen with the capture antibody on the magnetic particles. The supernatant was removed by attracting the magnetic particles with a magnet, and the magnetic particles were then washed twice with DPBS (200 µL). A TLip (DMPC) dispersion ([SQR23] = 1 µg/mL, 60 µL) carrying an influenza A detection antibody and a TLip (DPPC) dispersion ([SQR23] = 1 µg/mL, 60 µL) carrying a SARS-CoV-2 detection antibody were added to the washed magnetic particles to disperse the magnetic particles. The dispersion was then left standing at room temperature for 2 min to allow the TLip to bind to the antigen captured by the magnetic particles via the detection antibody. Then, the magnetic particles were attracted using a magnet to remove the TLiP that was not bound to the magnetic particles, and then the magnetic particles were washed twice with DPBS (200 µL). After adding DPBS (120 µL) to the well, the fluorescence intensity over time was measured using an optical fiber probe fluorescence detector (FLE 1100, manufactured by Nippon Sheet Glass Co., Ltd.) while heating the bottom surface of the well plate on a hot plate (65°C).

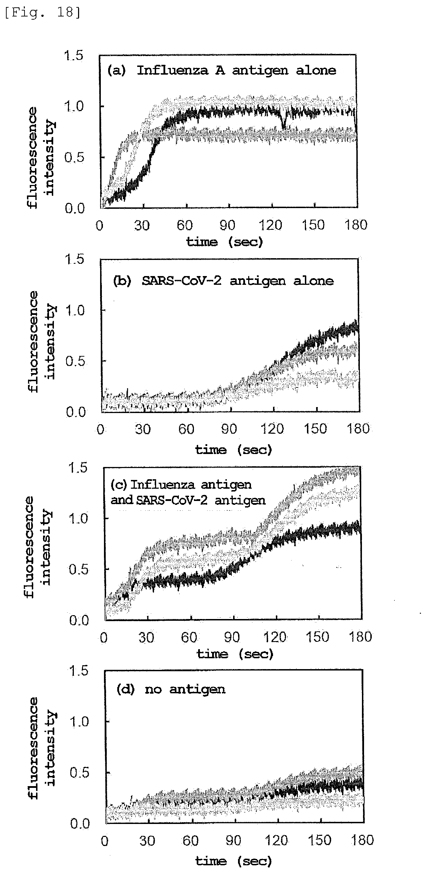

As shown in Fig. 18, in the system containing influenza A antigen alone, only an increase in fluorescence intensity due to the phase transition of TLip (DMPC) was observed immediately after the start of heating (Fig. 18a). Since TLip (DMPC) corresponds to the influenza A detection antibody, only influenza A antigen was detected. On the other hand, in the system containing SARS-CoV-2 antigen alone, no increase in fluorescence intensity was observed immediately after the start of heating, and after about 90 seconds, only an increase in fluorescence intensity due to the phase transition of TLip(DPPC), which corresponds to the SARS-CoV-2 detection antibody, was observed (Fig. 18b). Furthermore, in the system where both influenza A and SARS-CoV-2 antigens were added, the fluorescence intensity increased in two steps, derived from both TLip (DMPC) and TLip (DPPC), and both antigens of influenza A and SARS-CoV-2 were detected (Fig. 18c). In the system without antigen addition, no increase in the fluorescence intensity of either TLip (DMPC) or TLip (DPPC) was observed (Fig. 18d).

### (Example 21) Simultaneous detection of SARS-CoV-2 and influenza A N antigen (nucleocapsid protein)

30 µL of magnetic particles labeled with SARS-CoV-2 capture antibody (solid concentration: 10 µg/mL), 30 µL of magnetic particles labeled with influenza A capture antibody (solid concentration: 10 µg/mL), 30 µL of TLip (DPPC) dispersion ([SQR23] = 20 µg/mL) carrying SARS-CoV-2 detection antibody, and 30 µL of TLip(DMPC) dispersion ([SQR23] = 20 µg/mL) carrying influenza A detection antibody were mixed in a 384-well flat-bottom well. To this mixture was added SARS-CoV-2 recombinant N antigen at a final concentration of 100 ng/mL, influenza A recombinant N antigen (origin: Puerto Rico/8/1934 H1N1) was added at a final concentration of 0-400 ng/mL, and the mixture was left standing at room temperature for 5 min. In addition, influenza A recombinant N antigen was added at a final concentration of 200 ng/mL, and SARS-CoV-2 recombinant N antigen was added at final concentrations ranging from 0 to 75 ng/mL, and the mixture was left standing at room temperature for 5 min. The magnetic particles were then attracted using a magnet, the solution was removed, and the mixture was washed with PBS. The magnetic particles were then dispersed in 30 µL of PBS. The fluorescence intensity over time was measured using an optical fiber probe fluorescence detector (FLE 1100, manufactured by Nippon Sheet Glass Co., Ltd.) while heating the bottom surface of the well plate on a hot plate (70°C).

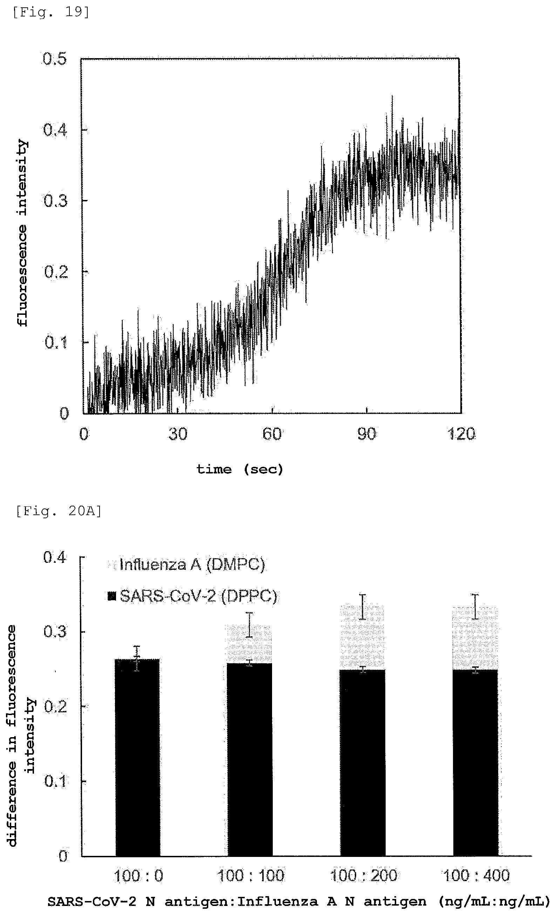

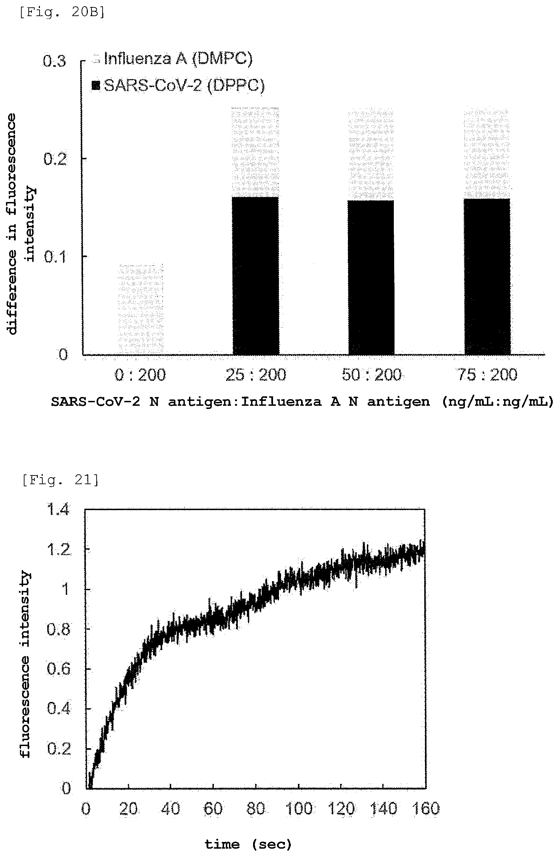

As shown in Fig. 19, when both SARS-CoV-2 and influenza A antigens were added, an increase in fluorescence intensity due to the phase transition of DMPC corresponding to influenza A was observed for about 50 seconds after the start of heating, followed by an increase in fluorescence intensity due to the phase transition of DPPC corresponding to SARS-CoV-2. As shown in Fig. 20A, when only SARS-CoV-2 antigen was added, there was no increase in fluorescence intensity until about 50 seconds after the start of heating, and only an increase in fluorescence intensity due to the phase transition of DPPC corresponding to SARS-CoV-2 was observed. As the influenza A antigen concentration increases, the fluorescence intensity difference due to the DMPC phase transition increases. In contrast, as shown in Fig. 20B, when influenza A antigen alone is added, there is no increase in fluorescence intensity after 50 seconds, and only an increase in fluorescence intensity due to the DMPC phase transition corresponding to influenza A is observed. When SARS-CoV-2 antigen was added, an increase in fluorescence intensity due to the phase transition of DPPC was observed after 50 seconds, indicating that SARS-CoV-2 was detected. For SARS-CoV-2, the difference in fluorescence intensity did not change even when the antigen concentration was changed from 25 to 75 ng/mL. This is considered to be because the antigen detection ability of the SARS-CoV-2 capture antibody and detection antibody pair (on the order of pg/mL) is significantly higher than that of the influenza antibody pair (on the order of ng/mL), and the antigen-antibody reaction has reached saturation in the antigen concentration range of ng/mL.

Then, using the microfluidic channel produced in Example 13, SARS-CoV-2 and influenza A were detected. Magnetic particles (solid concentration: 10 µg/mL) bound to each capture antibody were mixed with recombinant N antigen (influenza A = 200 ng/mL, SARS-CoV-2 = 25 ng/mL) and then introduced into the flow channel by using a syringe pump. After capturing the magnetic particles with a magnet, PBS (400 µL, 400 µL/min) was flowed through the flow channel to remove unbound recombinant N antigen. Then, the magnet capturing the magnetic particles was removed, and a mixed dispersion of TLip (DMPC) bound to an influenza A detection antibody ([SQR23]=5 µg/mL) and TLip (DPPC) bound to a SARS-CoV-2 detection antibody ([SQR23]=5 µg/mL was flowed through the flow channel at 400 µL/min, and the magnetic particles were captured by the magnet in the detection section. Then, PBS (400 µL) was flowed through the flow channel at 400 µL/min to wash out the unbound TLip, and fluorescence was detected using an optical fiber probe fluorescence detector (FLE 1100, manufactured by Nippon Sheet Glass Co., Ltd.) while heating the detection section on a hot plate (65°C). As shown in Fig. 21, an increase in fluorescence intensity due to the phase transition of DMPC (corresponding to influenza A antigen) was observed immediately after heating, and an increase in fluorescence intensity due to the phase transition of DPPC (corresponding to SARS-CoV-2 antigen) was observed from after 50 seconds. From the above results, it was shown that multiple antigens can be simultaneously tested based on the difference in the time until light emission, by loading antibodies corresponding to different antigens onto TLips with different phase transition temperatures.

### [Industrial Applicability]

According to the present invention, multiple target biomolecules contained in a biological sample can be simultaneously detected or quantified in a single assay, and a large amount of target biomolecules can be treated in a shorter time than before.

### (Reference documents)

1. Ji X., et al, Nanoscale, 2023, 15, 9290-9296.
2. Qi J., et al., Advanced Drug Delivery Reviews, 2019, 143, 206-225.
3. Zhang Y., et al., ACS Appl. Mater. Interfaces, 2013, 5, 8710-8717.
4. Hoekstra D. et al., Biochemistry, 1984, 23, 24, 5675-5681.
5. Somijit V. et al., Microporous and Mesoporous Materials, 2021, 328, 111452.
6. Tu L., et al., SmartMat., 2021, 2, 326-346.
7. Teixeira R. et al., Molecules, 2021, 26, 4264.
8. Reisch A. et al., Small, 2016, 12, 1968-1992.
9. BaumgartT. Et al., Biochim Biophys Acta 2007, 1768(9), 2182-2194.
10. Klymchenko A.S. et al., Chem Biol. 2014, 21(1), 97-113.
11. Filipe H. A. L. et al., Molecules 2020, 25, 3424.
12. Demchenko A. P. et al., Methods Mol Biol. 2015, 1232, 19-43.
13. Mei J. et al., Chem. Rev. 2015, 115, 21, 11718-11940.

## Claims

1. A method for detecting or quantifying a biomolecule in a biological sample, comprising the following steps (1) to (3):
(1) a step of bringing a magnetic particle bound to a biomolecular recognition element for capture, a biological sample, and a temperature-responsive fluorescent probe particle into contact with a magnetic substrate,
(2) a step of heating the magnetic substrate, and
(3) a step of measuring fluorescence emission, wherein
the temperature-responsive fluorescent probe particle is a temperature-responsive fluorescent particle bound to a biomolecular recognition element for detection,
the temperature-responsive fluorescent particle comprises at least one type of fluorescent molecule in a molecular assembly comprising and constituted by at least one type of amphiphilic lipid molecule,
the fluorescent molecule is quenched when the molecular assembly is in a solid phase, and emits fluorescence when the assembly is in a liquid phase, due to a temperature-responsive solid-liquid phase transition,
whereby quenching and fluorescence emission of the fluorescent molecule are reversibly switched in response to temperature,
the molecular assembly comprises aggregated fluorescent molecules when it is in a solid phase and dissociated fluorescent molecules when it is in a liquid phase, and
the fluorescent molecule is at least one compound selected from the group consisting of squaric acid derivatives, fluorescein derivatives, rhodamines, coumarins, oxazines, pyrenes, anthracenes, phthalocyanines, cyanines, azo dyes, carbopyronines, NBD derivatives, dansyl derivatives, BODIPY fluorophores, ALEXA fluorophores, rhodol dyes, naphthalimides, and porphyrins.

2. The method for detecting or quantifying a biomolecule according to claim 1, wherein the step (1) comprises the following steps:
(1') a step of bringing a magnetic particle bound to a biomolecular recognition element for capture into contact with a magnetic substrate,
(2') a step of contacting a biological sample with the magnetic particle, and
(3') a step of contacting a temperature-responsive fluorescent probe particle with the magnetic particle.

3. The method for detecting or quantifying a biomolecule according to claim 1, wherein the step (1) comprises the following steps:
(1'') a step of bringing a magnetic particle bound to a biomolecular recognition element for capture into contact with a biological sample,
(2") a step of contacting a complex obtained in step (1") with a magnetic substrate,
(3") a step of contacting a temperature-responsive fluorescent probe particle with the complex, and
(4") a step of contacting the complex obtained in step (3") with the magnetic substrate.

4. The method for detecting or quantifying a biomolecule according to claim 1, wherein the step (1) comprises the following steps:
(1‴) a step of contacting a temperature-responsive fluorescent probe particle with a biological sample,
(2‴) a step of contacting a complex obtained in step (1‴) with a magnetic particle bound to a biomolecular recognition element for capture, and
(3‴) a step of contacting the complex obtained in step (2‴) with a magnetic substrate.

5. The method for detecting or quantifying a biomolecule according to any one of claims 1 to 4, comprising a step of washing the magnetic particle between the steps (1) and (2).

6. The method for detecting or quantifying a biomolecule according to any one of claims 1 to 4, wherein the biomolecules to be detected or quantified are two or more types,
the magnetic particles are two or more types of magnetic particles bound to mutually different biomolecular recognition elements for capture, and
the temperature-responsive fluorescent probe particles are two or more types of temperature-responsive fluorescent particles bound to mutually different biomolecular recognition elements for detection and having different solid phase-liquid phase transition temperatures.

7. The method for detecting or quantifying a biomolecule according to any one of claims 1 to 4, wherein the molecular assembly has a lipid bilayer, and the fluorescent molecule is quenched when the molecular assembly is in a gel phase, and emits fluorescence when the assembly is in a liquid crystalline phase, due to a temperature-responsive gel-liquid crystalline phase transition,
whereby quenching and fluorescence emission of the fluorescent molecule are reversibly switched in response to temperature.

8. The method for detecting or quantifying a biomolecule according to any one of claims 1 to 4, wherein the amphiphilic molecule is a phospholipid.

9. The method for detecting or quantifying a biomolecule according to claim 8, wherein the molecular assembly is a bilayer membrane vesicle.

10. The method for detecting or quantifying a biomolecule according to claim 9, wherein the bilayer membrane vesicle comprises 0.3 mol % or more of a compound represented by the formula I.

11. The method for detecting or quantifying a biomolecule according to any one of claims 1 to 4, wherein the biomolecular recognition element is an antibody or a variable region of the antibody or a fab fragment of the antibody.

12. A kit for detecting or quantifying a biomolecule in a biological sample, comprising the following (1) and (2):
(1) a magnetic particle bound to a biomolecular recognition element for capture, and
(2) a temperature-responsive fluorescent probe particle which is a temperature-responsive fluorescent particle bound to a biomolecular recognition element for detection,
the temperature-responsive fluorescent particle comprises at least one type of fluorescent molecule in a molecular assembly comprising and constituted by at least one type of amphiphilic lipid molecule,
the fluorescent molecule is quenched when the molecular assembly is in a solid phase, and emits fluorescence when the assembly is in a liquid phase, due to a temperature-responsive solid-liquid phase transition,
whereby quenching and fluorescence emission of the fluorescent molecule are reversibly switched in response to temperature,
the molecular assembly comprises aggregated fluorescent molecules when it is in a solid phase and dissociated fluorescent molecules when it is in a liquid phase, and
the fluorescent molecule is at least one compound selected from the group consisting of squaric acid derivatives, fluorescein derivatives, rhodamines, coumarins, oxazines, pyrenes, anthracenes, phthalocyanines, cyanines, azo dyes, carbopyronines, NBD derivatives, dansyl derivatives, BODIPY fluorophores, ALEXA fluorophores, rhodol dyes, naphthalimides, and porphyrins.

13. The kit for detecting or quantifying a biomolecule according to claim 12, further comprising a microfluidic chip.
